# EUROPÄISCHE PATENTSCHRIFT

(11) **EP 1 105 371 B1**
(45) Veröffentlichungstag und Bekanntmachung des Hinweises auf die Patenterteilung: **03.11.2004**
(21) Anmeldenummer: 99940158.1
(22) Anmeldetag: 06.08.1999
(51) Int. Cl.: C07C 309/65, A61K 31/255

(54) **AMINOSÄUREESTER VON ARYLSULFONAMIDEN UND ANALOGA**
NOVEL ARYL SULPHONAMIDE AMINO ACID ESTERS AND ANALOGUES
NOUVEAUX ESTERS D'AMINOACIDES D'ARYLSULFONAMIDES ET LEURS ANALOGUES

(30) Priorität: 19.08.1998 DE 19837627
(43) Veröffentlichungstag der Anmeldung: 13.06.2001
(73) Patentinhaber: Bayer HealthCare AG, 51368 Leverkusen (DE)
(72) Erfinder: MITTENDORF, Joachim, D-42113 Wuppertal (DE); DRESSEL, Jürgen, D-42477 Radevormwald (DE); MATZKE, Michael, D-42113 Wuppertal (DE); KELDENICH, Jörg, D-42113 Wuppertal (DE); MAULER, Frank, D-51491 Overath (DE); DE VRY, Jean-Marie, Victor, D-51503 Rösrath (DE); FRANZ, Jürgen, D-58456 Witten (DE); SPREYER, Peter, D-40225 Düsseldorf (DE); VÖHRINGER, Verena, D-42113 Wuppertal (DE); SCHUMACHER, Joachim, D-42113 Wuppertal (DE); ROCK, Michael-Harold, DK-2650 Hvidovre (DK); HORVATH, Ervin, D-51373 Leverkusen (DE); FRIEDL, Arno, D-51427 Bergisch Gladbach (DE)
(86) Internationale Anmeldenummer: PCT/EP1999/005683
(87) Internationale Veröffentlichungsnummer: WO 2000/010968

(56) Entgegenhaltungen:
- WO-A-91/14674
- WO-A-98/37061
- FR-A- 2 735 774
- US-A- 4 243 819

## Beschreibung

Die vorliegende Erfindung betrifft neue Aminosäureester von Arylsulfonamiden und Analoga, Verfahren zu ihrer Herstellung und ihre Verwendung zur Prophylaxe und Behandlung von neurodegenerativen Erkrankungen, insbesondere zur Behandlung von Apoplexia Cerebri, Schädel-Hirn-Trauma, Schmerz und Spastizität.

Δ⁹-Tetrahydrocannabinol (Δ⁹-THC) und in geringem Maße auch Δ⁸-THC sind die biologisch aktiven Bestandteile in Extrakten der Pflanze Cannabis sativa (Marihuana, Haschisch) und sind verantwortlich für die Effekte auf das menschliche Zentrale Nervensystem (ZNS). Potentielle historische und kontemporäre therapeutische Anwendungen von Cannabis-Präparaten umfassen u.a. Analgesie, Emesis, Anorexie, Glaukom und Bewegungsstörungen.

Bislang wurden zwei Subtypen von Cannabinoid-Rezeptoren und eine Spleiß-Variante identifiziert. Der CB1-Rezeptor (Nature 1990, 346, 561) und eine Spleiß-Variante CB1a (J. Biol. Chem. 1995, 270, 3726) sind überwiegend im Zentralen Nervensystem lokalisiert. Der CB2-Rezeptor wurde überwiegend im peripheren Gewebe, insbesondere in Leukozyten, Milz und Makrophagen gefunden (Eur. J. Biochem. 1995, 232, 54).

CB1 und CB2-Rezeptoren besitzen sieben Transmembranregionen und gehören zur Familie der G-Protein-Rezeptoren. Beide Rezeptoren sind negativ gekoppelt via Gᵢ/Gₒ-Protein zur Adenylatcyclase und möglicherweise negativ gekoppelt zur präsynaptischen Freisetzung von Glutamat (J. Neurosci. 1996, 16, 4322). CB1-Rezeptoren sind darüberhinaus positiv gekoppelt mit Kalium-Kanälen sowie negativ gekoppelt mit N- und Q-Typ Calcium-Kanälen.

Vier Klassen von CB1-Rezeptor-Agonisten sind bisher bekannt: klassische Cannabinoide, wie beispielsweise Δ⁹-THC, nichtklassische Cannabinoide, Aminoalkylindole und Eicosanoide. Zu den letzten gehört der allgemein akzeptierte endogene CB1-Rezeptor-Agonist Anandamid.

Wenige spezifische CB2-Rezeptor-Agonisten sind bekannt, wie z.B. aus der FR-A-2 765 774.

Außerdem ist bekannt, daß Apoplexia Cerebri eine Folge einer plötzlichen Durchblutungsstörung eines menschlichen Gehirnbereichs mit nachfolgenden Funktionsausfällen, mit entsprechenden neurologischen und/oder psychischen Symptomen ist. Die Ursachen für Apoplexia Cerebri können in Hirnblutungen (z.B nach einem Gefäßriß bei Hypertonie, Arteriosklerose und apoplektischem Aneurysma) und Ischämien (z.B. durch eine Blutdruckabfallkrise oder Embolie) liegen. Die Funktionsausfälle im Gehirn führen zu einer Degeneration oder Abtötung der Gehirnzellen (Journal of Cerebral Blood Flow and Metabolism 1981, 1, 155); Chem. Eng. News 1996 (May 13), 41; Trends Pharmacol. Sci. 1996, 17, 227). Unter Schädel/Him-Trauma versteht man gedeckte und offene Schädelverletzungen mit Gehimbeteiligung.

Die vorliegende Erfindung betrifft Verbindungen der allgemeinen Formel (I)

R¹-A-D-E-G-L-R² (I)

in welcher
- R¹: für einen Rest der Formel 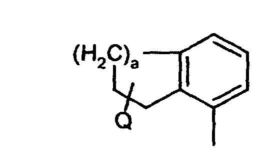 steht,
worin
- a: eine Zahl 1 oder 2 bedeutet,
und wobei der oben aufgeführte Phenylrest gegebenenfalls mit einem oder mehreren, gleichen oder verschiedenen Substituenten substituiert ist, die aus der Gruppe ausgewählt sind, die besteht aus:
Chlor, Fluor Hydroxy, (C₁-C₃) Alkoxy, (C₁-C₄)-Alkyl, das seinerseits durch Hydroxy substituiert sein kann,
- Q: einen Rest der Formel 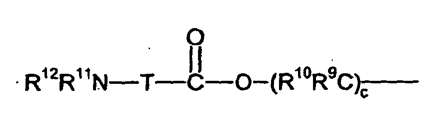 bedeutet,
worin
c eine Zahl 1, 2, 3 oder 4 bedeutet,
R⁹ und R¹⁰ gleich oder verschieden sind und Wasserstoff oder (C₁-C₃)-Alkyl bedeuten,
T einen Rest der Formel -(CH₂)_{d}- bedeutet, worin
d eine Zahl 1, 2, 3, 4, 5 oder 6 bedeutet, oder
T einen Teil eines Aminosäurerestes der Formel 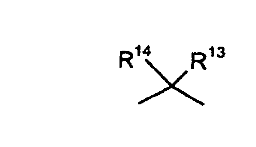 bedeutet, worin
R¹³ Wasserstoff oder Methyl bedeutet, und
R¹⁴ Cyclopentyl, Cyclohexyl, Phenyl oder Wasserstoff bedeutet, oder
(C₁-C₄)-Alkyl bedeutet, wobei das (C₁-C₄)-Alkyl gegebenenfalls durch Methlythio, Hydroxy, Mercapto, Guanidyl oder durch eine Gruppe der Formel -NR¹⁵R¹⁶ substituiert ist,
worin
R¹⁵ und R¹⁶ unabhängig voneinander Wasserstoff, (C₁-C₈)-Alkyl oder Phenyl bedeuten oder das (C₁-C₄)-Alkyl gegebenenfalls durch Cyclopentyl, Cyclohexyl oder Phenyl substituiert ist, das seinerseits durch Hydroxy, Fluor, Chlor oder (C₁-C₃)-Alkoxy oder Amino substituiert ist,
und
R¹¹ und R¹² gleich oder verschieden sind und Wasserstoff oder (C₁-C₆)-Alkyl bedeuten, oder
R¹¹ und R¹² gemeinsam mit dem Stickstoffatom einen Morpholinring bilden,
- A und E: für eine Bindung stehen,
- D: für ein Sauerstoffatom steht,
- G: für zweifach gebundenes Phenyl steht, das gegebenenfalls mit einem oder mehreren, gleichen oder verschiedenen Substituenten substituiert ist die aus der Gruppe ausgewählt sind, die besteht aus:
Hydroxy, Trifluormethyl, Carboxyl, Fluor, Chlor, Brom (C₁-C₃)-Alkyl, Hydroxy(C₁-C₃)alkyl oder (C₁-C₃)-Alkoxy,
- L: für einen Rest der Formel 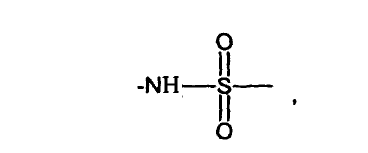 oder 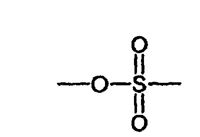 steht
wobei die Anbindung der Reste an G linksbündig erfolgt,
- R²: für
(C₁-C₈)-Alkyl, das gegebenenfalls mit einem oder mehreren, gleichen oder verschiedenen Substituenten substituiert ist, die aus der Gruppe ausgewählt sind, die besteht aus:
Fluor, Chlor, Brom, Phenyl, Trifluormethyl oder Trifluormethyl substituierten (C₁-C₄)-Alkoxy,
   und deren pharmazeutisch verträgliche Salze.

Aminoschutzgruppe im Rahmen der Erfindung sind die üblichen in der Peptid--Chemie verwendeten Aminoschutzgruppen.
Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert.Butoxycarbonyl, Allyloxycarbonyl, Vinyloxycarbonyl, 2-Nitrobenzyloxycarbonyl, 3,4,5-Trimethoxybenzyloxycarbonyl, Cyclohexoxycarbonyl, 1,1-Dimethylethoxycarbonyl, Adamantylcarbonyl, Phthaloyl, 2,2,2-Trichlorethoxycarbonyl, 2,2,2-Trichlor-tert-butoxycarbonyl, Menthyloxycarbonyl, Phenoxycarbonyl, 4-Nitrophenoxycarbonyl, Fluorenyl-9-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl oder 4-Nitrophenyl.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Diese Mischungen der Enantiomeren und Diastereomeren lassen sich in bekannter Weise in die stereoisomer einheitlichen Bestandteile trennen.

Die erfindungsgemäßen Verbindungen können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Zur vorliegenden Erfindung gehören auch Ammoniumverbindungen, die durch Überführung der freien Amine mittels Alkylierung hergestellt werden können.

Im Rahmen der vorliegenden Erfindung haben die Substituenten im allgemeinen die folgende Bedeutung:
(C₁-C₁₂)-Alkyl steht im allgemeinen in Abhängigkeit von den oben aufgeführten Substituenten für einen geradkettigen oder verzweigten Kohlenwassserstoffrest mit 1 bis 12 Kohlenstoffatomen. Beispielsweise seien Methyl, Ethyl, Propyl, Isopropyl, Butyl, Isobutyl, Pentyl, Isopentyl, Hexyl, Isohexyl, Heptyl, Isoheptyl, Octyl und Isooctyl genannt. Bevorzugt ist (C₁-C₈)-Alkyl mit 1 bis 8 Kohlenstoffatomen, z.B. Methyl, Ethyl, Propyl oder Isopropyl.
(C₂-C₁₂)-Alkenyl stehen im allgemeinen in Abhängigkeit von den oben aufgeführten Substitutenten für einen geradkettigen oder verzweigten Kohlenwasserstoffrest mit 2 bis 6 und 2 bis 20 Kohlenstoffatomen und einer oder mehreren, bevorzugt mit einer oder zwei Doppelbindungen. Bevorzugt ist der Niederalkylrest mit 2 bis 4 und 2 bis 10 Kohlenstoffatomen und einer Doppelbindung. Besonders bevorzugt ist ein Alkenylrest mit 2 bis 3 und 2 bis 8 Kohlenstoffatomen und einer Doppelbindung. Beispielsweise seien Allyl, Propenyl, Isopropenyl, Butenyl, Isobutenyl, Pentenyl, Isopentenyl, Hexenyl, Isohexenyl, Heptenyl, Isoheptenyl, Octenyl und Isooctenyl genannt.
(C₂-C₁₂)-Alkinyl steht im allgemeinen in Abhängigkeit von den oben aufgeführten Substituenten für einen geradkettigen oder verzweigten Kohlenwasserstoffrest mit 2 bis 12 Kohlenstoffatomen und einer oder mehreren, bevorzugt mit einer oder zwei Dreifachbindungen. Bevorzugt ist der Niederalkylrest mit 2 bis etwa 10 Kohlenstoffatomen und einer Dreifachbindung. Besonders bevorzugt ist ein Alkylrest mit 2 bis 8 Kohlenstoffatomen und einer Dreifachbindung. Beispielsweise seien Acetylen, 2-Butin, 2-Pentin und 2-Hexin genannt.
(C₁-C₆)-Acyl steht im allgemeinen in Abhängigkeit von den oben aufgeführten Substituenten für geradkettiges oder verzweigtes Niedrigalkyl mit 1 bis 6 Kohlenstoffatomen, die über eine Carbonylgruppe gebunden sind. Bevorzugt sind Alkylreste mit bis zu 4 Kohlenstoffatomen. Ganz besonders bevorzugt sind beispielsweise Alkylreste mit bis zu 3 Kohlenstoffatomen. Beispielsweise seien genannt: Acetyl, Ethylcarbonyl, Propylcarbonyl, Isopropylcarbonyl, Butylcarbonyl und Isobutylcarbonyl.
(C₁-C₆)-Alkoxy steht im allgemeinen in Abhängigkeit von den oben aufgeführten Substituenten für einen über ein Sauerstoffatom gebundenen geradkettigen oder verzweigten Kohlenwasserstoffrest mit 1 bis 6 Kohlenstoffatomen. Bevorzugt ist Niederalkoxy mit 1 bis 4 Kohlenstoffatomen. Besonders bevorzugt ist ein Alkoxyrest mit 1 bis 3 Kohlenstoffatomen. Beispielsweise seien Methoxy, Ethoxy, Propoxy, Isopropoxy, Butoxy, Isobutoxy, Pentoxy, Isopentoxy, Hexoxy, Isohexoxy genannt.
(C₁-C₆)- Alkoxycarbonyl kann beispielsweise durch die Formel 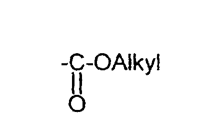 dargestellt werden.
Alkyl steht hierbei für einen geradkettigen oder verzweigten Kohlenwasserstoffrest mit 1 bis 6 Kohlenstoffatomen. Bevorzugt wird Niederalkoxycabonyl mit 1 bis 4 Kohlenstoffatomen im Alkylteil. Beispielsweise seien die folgenden Alkoxycarbonylreste genannt: Methoxycarbonyl, Ethoxycarbonyl, Propoxycarbonyl, Isopropoxycarbonyl, Butoxycarbonyl oder Isobutoxycarbonyl.
(C₃-C₈)-Cycloalkyl steht im allgemeinen für einen cyclischen Kohlenwasserstoffrest mit 3 bis 8 Kohlenstoffatomen. Bevorzugt sind Cyclopropyl, Cyclopentyl und Cyclohexyl. Beispielsweise seien Cyclopentyl, Cyclohexyl, Cycloheptyl und Cyclooctyl genannt.
cyclo(C₄-C₇)Acyl steht im allgemeinen in Abhängigkeit von den oben aufgeführten Substituenten für Cyclopropylcarbonyl, Cyclobutylcarbonyl, Cyclopentylcarbonyl oder Cyclohexylcarbonyl.
(C₆-C₁₀)-Aryl steht im allgemeinen für einen aromatischen Rest mit 6 bis 10 Kohlenstoffatomen. Bevorzugte Arylreste sind Phenyl und Naphthyl.
(C₁-C₆)-Perfluoralkoxy steht im Rahmen der Erfindung für einen Alkoxyrest mit 1 bis 6 Kohlenstoffatomen und 3 bis 13 Fluoratomen. Bevorzugt ist ein Alkoxyrest mit 1 bis 5 Kohlenstoffatomen und 3 bis 9 Fluoratomen.
(C₁-C₆)-partiell fluoriertes Alkoxy steht im Rahmen der Erfindung für einen Alkoxyrest mit 1 bis 6 Kohlenstoffatomen und 3 bis 5 Fluoratomen. Bevorzugt ist ein Alkoxyrest mit 1 bis 4 Kohlenstoffatomen und 3 Fluoratomen. Besonders bevorzugt ist ein Alkoxyrest mit 1 bis 3 Kohlenstoffatomen, der durch Trifluormethyl substituiert ist.
Halogen steht im Rahmen der Erfindung für Fluor, Chlor, Brom und Jod.
Aromatische, gesättigte und ungesättigte Heterocyclen stehen im Rahmen der Erfindung in Abhängigkeit von den oben aufgeführten Substituenten im allgemeinen für einen 5- bis 7-gliedrigen oder 5- bis 6-gliedrigen, vorzugsweise 5- bis 6-gliedrigen Heterocyclus, der bis zu 3 Heteroatome aus der Reihe S, N und/oder O enthalten und der gegebenenfalls auch über ein Stickstoffatom gebunden sein kann. Beispielsweise seien genannt: Pyridyl, Thienyl, Furyl, Pyrrolyl, Pyrrolidinyl, Piperazinyl, Pyrimidyl, Thiazolyl, Oxazolyl, Imidazolyl, Morpholin oder Piperidyl. Bevorzugt sind Pyridyl, Furyl, Morpholin, Piperidyl und Piperazinyl.
Abgangsgruppen im Sinne der Erfindung sind Gruppen, die in einer nukleophilen Substitution durch ein Nukleophil ersetzt werden können (Streitwieser, A., Jr.; Heathcock, C.H. Organische Chemie, Verlag Chemie, 1980, S. 169ff.). Bevorzugte Abgangsgruppen sind Halogenide und Sulfonsäureester/-anhydride. Eine besonders bevorzugte Abgangsgruppe ist Chlorid.
(C₃-C₆)-Keton steht im Rahmen der Erfindung für ein gesättigtes oder ungesättigtes Keton mit 3 bis 6 Kohlenstoffatomen. Beispielsweise seien genannt: Aceton, Butanon, But-1-en-3-on, But-1-in-3-on, Pentan-3-on, Pentan-2-on, Pent-1-en-3-on, Pent-1-in-3-on, Penta-1,4-dien-3-on, 3-Methylbutan-2-on, Cyclopropylmethylketon, Cyclopentanon, Hexan-2-on, Hexan-3-on, Cyclohexanon, 2-Methylcyclopentanon, 2-Ethylcyclobutanon.
(C₁-C₆)-Aldehyd steht im Rahmen der Erfindung für einen gesättigten oder ungesättigten Aldehyd mit 1 bis 6 Kohlenstoffatomen. Beispielsweise seien genannt: Formaldehyd, Acetaldehyd, Propionaldehyd, Butyraldehyd, Isobutyraldehyd, Cyclopropylcarbaldehyd, But-2-enal, But-2-inal, Pentanal, Isopentanal, Pivaldehyd, Cyclobutylcarbaldehyd, 2-Methylcyclopropylcarbaldehyd, Pent-2-enal, Pent-4-enal, Hexanal, 2-Cyclobutylacetaldehyd.

Ganz besonders bevorzugt sind erfindungsgemäße Verbindungen der Formel (I),
worin
- R¹: für einen Rest der Formel 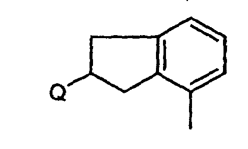 steht,
worin
Q einen Rest der Formel 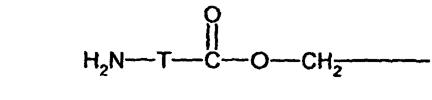 bedeutet, worin
T einen Rest der Formel -(CH₂)_{d}- bedeutet, worin
d eine Zahl 1, 2, 3, 4, 5 oder 6 bedeutet,
oder
T einen Teil eines Aminosäurerestes der Formel 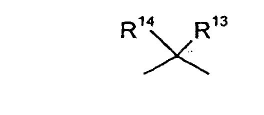 bedeutet, worin
R¹³ Wasserstoff bedeutet, und
R¹⁴ Wasserstoff, (C₁-C₄)-Alkyl, Benzyl oder einen Rest der Formel -CH₂OH bedeutet,
- A und E: für eine Bindung stehen,
- D: für ein Sauerstoffatom steht,
- G: für Phenyl steht, das gegebenenfalls durch Fluor, Chlor oder Brom substituiert ist,
- L: für einen Rest der Formel 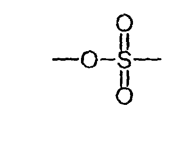 steht,
wobei die Anbindung des Restes an G linksbündig erfolgt,
- R²: für (C₁-C₄)-Alkyl steht, das gegebenenfalls durch Fluor oder Trifluormethyl substituiert ist,
und deren pharmazeutisch verträgliche Salze.

Ebenso besonders bevorzugt sind Verbindungen ausgewählt aus der Gruppe
(R)-4,4,4-Trifluor-1-butansulfonsäure-3-(2-glycinyl-oxymethyl-indanyl-4-oxy)-phenylester 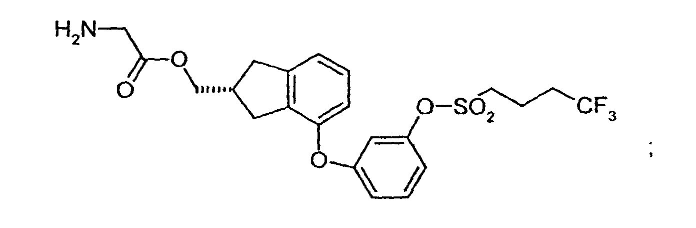 (R)-4,4,4-Trifluor-1-butansulfonsäure-3-[2-(7-aminoheptanoyloxymethyl)-indanyl-4-oxy]-phenylester 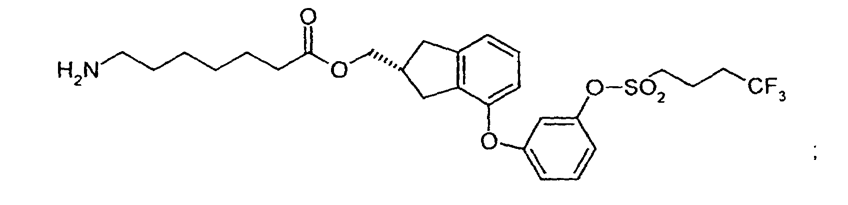 (R)-4,4,4-Trifluor-1-butansulfonsäure-3-[2-(3-aminopropanoyloxymethyl)-indanyl-4-oxy]-phenylester 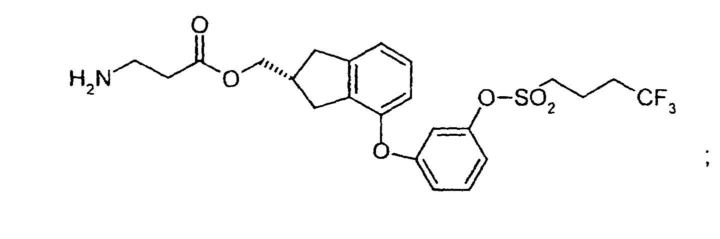 und
(R)-4,4,4-Trifluor-1-butansulfonsäure-3-[2-((S)-valinyloxymethyl)-indanyl-4-oxy]-phenylester 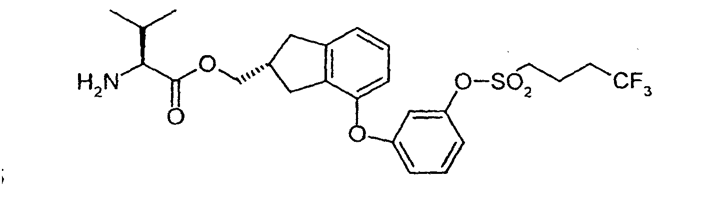 und deren pharmazeutisch verträgliche Salze.

Außerdem wurde ein Verfahren zur Herstellung der erfindungsgemäßen Verbindungen der allgemeinen Formel (I) gefunden, dadurch gekennzeichnet, daß man Verbindungen der allgemeinen Formel (II)

R^{1'}-A-D-E-G-L-R² (II)

worin
- A, D,E, G, L, R²,: die oben angegebene Bedeutung haben
und
- R^{1'}: für einen Rest der Formel 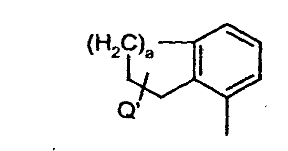 steht,
worin
- a: eine Zahl 1 oder 2 bedeutet,
und wobei der oben aufgeführte Phenylrest, gegebenenfalls mit einem oder mehreren, gleichen oder verschiedenen Substituenten substituiert ist, die aus der Gruppe ausgewählt sind, die besteht aus:
Chlor, Fluor, Hydroxy, Phenyl, (C₁-C₃)-Alkoxy, (C₁-C₄)-Alkyl, das seinerseits durch Hydroxy substituiert sein kann,
   und
   Q' einen Rest der Formel HO-(R¹⁰R⁹C)_{c})- bedeutet,
   worin
   c, R⁹ und R¹⁰ die oben angegebene Bedeutung haben,
mit Verbindungen der allgemeinen Formel (III) 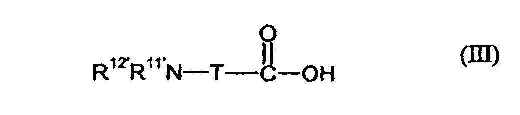 in welcher
R^{11'} für Wasserstoff steht und
R^{12'} für eine der oben aufgeführten Aminoschutzgruppen, vorzugsweise für tert.Butyloxycarbonyl steht,
R^{12'} für eine der oben aufgeführten Aminoschutzgruppen, vorzugsweise für tert.Butyloxycarbonyl steht,
in inerten Lösemitteln, gegebenenfalls in Anwesenheit einer Base und eines Hilfsstoffes,
umsetzt,
und die Aminoschutzgruppe nach üblichen Methoden abspaltet,
und dann gegebenenfalls die Aminogruppe mit einem Aldehyd oder Keton reduktiv alkyliert oder dialkyliert,
oder mit einem Halogenid alkyliert oder dialkyliert,
und gegebenenfalls in Abhängigkeit der oben aufgeführten Substituenten nach üblichen Methoden wie beispielsweise einer Alkylierung oder Veresterung Derivatisierungen anschließt.

Das erfindungsgemäße Verfahren kann durch folgendes Formelschema beispielhaft erläutert werden:

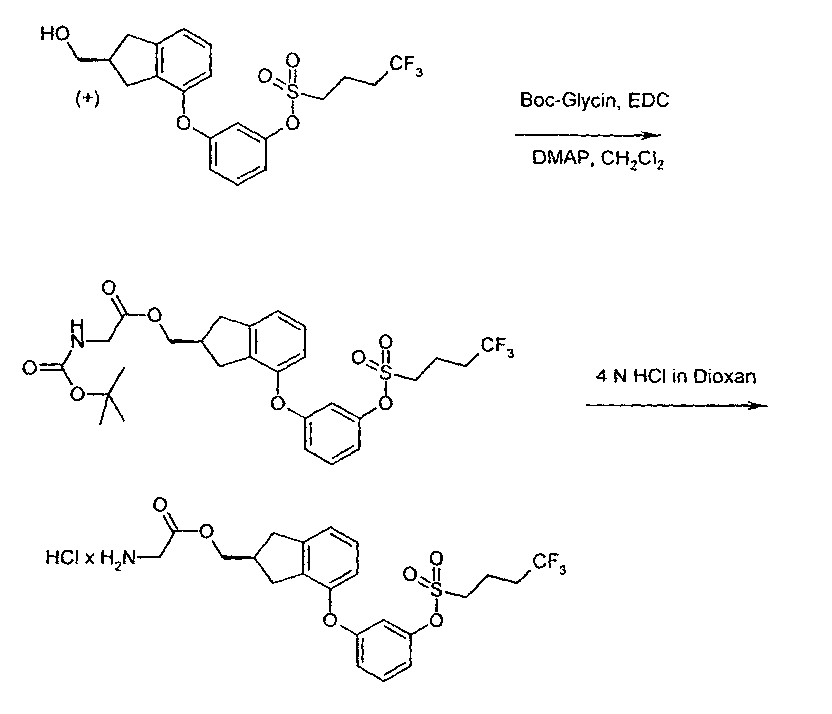

Als Lösemittel eignen sich für alle Verfahrensschritte die üblichen inerten Lösemittel, die sich unter den Reaktionsbedindungen nicht verändern. Hierzu gehören bevorzugt organische Lösemittel wie Ether z.B. Diethylether, Glykolmono- oder -dimethylether, Dioxan oder Tetrahydrofuran, oder Kohlenwasserstoffe wie Benzol, p-Kresol, Toluol, Xylol, Cyclohexan oder Erdölfraktionen oder Halogenkohlenwasserstoffe wie Methylenchlorid, Chloroform, Tetrachlorkohlenstoff, oder Dimethylsulfoxid, Dimethylformamid, Hexamethylphosphorsäuretriamid, Essigester, Pyridin, Triethylamin oder Picolin. Ebenso ist es möglich, Gemische der genannten Lösemittel, gegebenenfalls auch mit Wasser zu verwenden. Besonders bevorzugt sind Methylenchlorid, Tetrahydrofuran, Dioxan und Dioxan/Wasser.

Als Basen eignen sich organische Amine-(C₁-C₆)-Trialkylamine wie beispielsweise Triethylamin oder Heterocyclen wie Pyridin, Methylpiperidin, Piperidin oder N-Methylmorpholin. Bevorzugt sind Triethylamin und N-Methylmorpholin.

Die Basen werden im allgemeinen in einer Menge von 0,1 mol bis 5 mol, bevorzugt von 1 mol bis 3 mol jeweils bezogen auf 1 mol der Verbindungen der allgemeinen Formel (II) eingesetzt.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Die Reaktionen werden in einem Temperaturbereich von 0° C bis 100°C, vorzugsweise bei 0°C bis 30°C und bei Normaldruck durchgeführt.

Die Abspaltung der Aminoschutzgruppen erfolgt in an sich bekannter Weise.

Als Hilfsstoffe für die jeweiligen Peptidkupplungen werden bevorzugt Kondensationsmittel eingesetzt, die auch Basen sein können, insbesondere wenn die Carboxylgruppe als Anhydrid aktiviert vorliegt. Bevorzugt werden hier die üblichen Kondensationsmittel wie Carbodiimide z.B. N,N'-Diethyl-, N,'-Dipropyl-, n;n'-Diisopropyl-, N,N'-Dicyclohexylcarbodiimid, N-(3-Dimethylaminoisopropyl)-N'-ethylcarbodiimid-Hydrochlorid, oder Carbonylverbindungen wie Carbonyldiimidazol, oder 1,2-Oxazoliumverbindungen wie 2-Ethyl-5-phenyl-1,2―oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphobat, oder 1-Hydroxybenzotriazol und als Basen Alkalicarbonate z.B. Natrium- oder Kaliumcarbonat, oder -hydrogencarbonat, oder organische Basen wie Trialkylamine z.B. Triethylamin, N-Ethylmorpholin, N-Methylpiperidin oder Diisopropylethylamin eingesetzt. Besonders bevorzugt sind Dicyclohexylcarbodiimid, N-Methylmorpholin und 1-Hydroxybenztriazol.

Die Alkylierung erfolgt im allgemeinen mit Alkylierungsmitteln wie beispielsweise Alkylhalogenide, Sulfonsäureester oder substituierte oder unsubstituierte Dialkyl- oder Diarylsulfonate, vorzugsweise mit Methyljodid oder Dimethylsulfat.

Die Alkylierung erfolgt im allgemeinen in einem der oben aufgeführten Lösemitteln, vorzugsweise in Dimethylformamid in einem Temperaturbereich von 0°C bis +70°C, vorzugsweise von 0°C bis +30°C und Normaldruck.

Die Verbindungen der allgemeinen Formel (III) sind an sich bekannt oder nach üblichen Methoden herstellbar.

Die Verbindungen der allgemeinen Formel (II) können hergestellt werden, indem man
[A] Verbindungen der allgemeinen Formel (IV)

   R^{1"}-A-D-E-G-M-H (IV)

   in welcher
   - R^{1"}: die oben angegebene Bedeutung von R^{1'} hat , aber statt Q' der Substituent Q" eingeführt wird,
   worin Q" für eine Gruppe der Formel (C₁-C₃)-Alkyl-O₂C-(R¹⁰R⁹C)_{c"} bedeutet,
   worin c" eine Zahl 0, 1, 2, 3, 4 oder 5 bedeutet,
   und
   R⁹ und R¹⁰ die oben angegebene Bedeutung haben,
   - A, D, E und G: die oben angegebene Bedeutung haben
   und
   - M: für Sauerstoff oder -N(R⁴²)- steht,
   worin
   R⁴² Wasserstoff oder (C₁-C₄)-Alkyl ist,
   mit Verbindungen der allgemeinen Formel (V)

   R⁴³-W-R² (V)

   in welcher
   - R²: die oben angegebene Bedeutung hat,
   - R⁴³: für Halogen, vorzugsweise Chlor oder Iod steht,
   - W: für einen Rest der Formel -SO₂-, -SO-, -CO- , -P(O)(OR³⁷)- oder eine Einfachbindung steht,
   worin
   R³⁷ die oben angegebene Bedeutung hat,
   zu Verbindungen der allgemeinen Formel (V')

   R^{1"}-A-D-E-G-M-W-R² (V')

   in welcher
   - R^{1"}, A, D, E, G, M. W und R²: die oben angegebene Bedeutung haben,
   in inerten Lösemitteln, gegebenenfalls in Anwesenheit einer Base, umgesetzt werden,
   und abschließend zu Verbindungen der allgemeinen Formel (IIa)

   R^{1"}A-D-E-G-M-W-R² (IIa)

   in welcher
   - R^{1'}, A, D, E, G, M, W und R²: die oben angegebene Bedeutung haben,
   reduziert,
   oder
[B] Verbindungen der allgemeinen Formel (IV)
   zunächst mit Chlorsulfonsäuretrialkylsilylester, vorzugsweise Chlorsulfonsäuretrimethylsilylester, umgesetzt werden, mit einer Säure versetzt werden und dann mit einem Chlorierungsmittel, vorzugsweise Phosphorpentachlorid, zu einer Verbindung der allgemeinen Formel (VI)

   R^{1"}-A-D-E-G-M-SO₂-Cl (VI)

   in welcher
   - R^{1"}, A, D, E, G und M: die oben angegebene Bedeutung haben,
   umgesetzt werden und anschließend mit Verbindungen der allgemeinen Formel (VII)

   H-X-R² (VII)

   in welcher
   - R²: die im Anspruch 1 angegebene Bedeutung hat und
   - X: für Sauerstoff oder Stickstoff steht,
   zu Verbindungen der allgemeinen Formel (VII')

   R^{1"}-A-D-E-G-M-SO₂-X-R² (VII')

   in welcher
   - R^{1"}, A, D, E, G, M, X und R²: die oben angegebene Bedeutung haben,
   in inerten Lösemitteln in Anwesenheit von Bzl-NEt₃⁺Cl⁻ und einer Base, umgesetzt werden,
   und abschließend zu Verbindungen der allgemeinen Formel (IIb)

   R^{1'}-A-D-E-G-M-SO₂-X-R² (IIb)

   in welcher
   - R^{1'}, A, D, E, G, M, X und R²: die oben angegebene Bedeutung haben,
   reduziert,
   oder
[C] Verbindungen der allgemeinen Formel (VIII)

   R^{1"}-A-D'-H (VIII)

   in welcher
   - R^{1"} und A: die oben angegebene Bedeutung haben und
   - D': für Sauerstoff, Schwefel oder -N(R¹⁹)- steht
   worin
   R¹⁹ die in Anspruch 1 angegebene Bedeutung hat,
   mit Verbindungen der allgemeinen Formel (IX)

   R⁴⁴-E-G-SO₂-NH-R² (IX)

   in welcher
   - E, G und R²: die oben angegebene Bedeutung haben und
   - R⁴⁴: für eine Abgangsgruppe, bevorzugt Halogen, besonders bevorzugt Fluor, Chlor oder Brom steht,
   zu Verbindungen der allgemeinen Formel (IX')

   R^{1"}-A-D'-E-G-SO₂-NH-R² (IX')

   in welcher
   - R^{1"}, A, D', E, G und R²: die oben angegebene Bedeutung haben,
   umgesetzt werden, und abschließend zu Verbindungen der allgemeinen Formel (IIc)

   R^{1'}-A-D'-E-G-SO₂-NH-R² (IIc)

   in welcher
   - R^{1'}, A, D', E, G und R²: die oben angegebene Bedeutung haben,
   reduziert,
   oder
[G] Verbindungen der allgemeinen Formel (IIh) 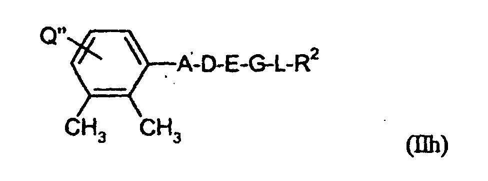 in welcher
   - Q", A,D,E,G,L und R²: die oben angegebene Bedeutung haben,
   durch radikalische Bromierung, beispielsweise mit N-Bromsuccinimid, in einem inerten Lösungsmittel in Verbindungen der allgemeinen Formel (IIi) 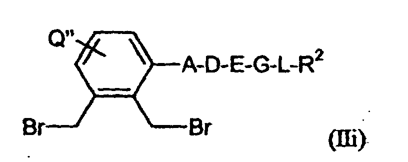 in welcher
   - Q", A, D, E, G, L und R²: die oben angegebene Bedeutung haben,
   übergeführt werden,
   und anschließend mit Verbindungen der Formel (XI)

   CH₂(CO₂R⁵²)₂ (XI)

   in welcher
   - R⁵²: für (C₁-C₆)-Alkyl steht,
   in inerten Lösemitteln, gegebenenfalls in Anwesenheit einer Base, zu Verbindungen der allgemeinen Formel (XIT)

   R⁵³-A-D-E-G-L-R² (XII')

   in welcher
   - A, D, E, G, L und R²: die oben genannte Bedeutung haben und
   - R⁵³: für 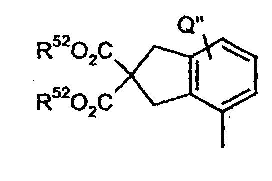 steht,
   worin
   Q", R⁵² die oben genannte Bedeutung haben,
   umgesetzt werden,
   und abschließend zu Verbindungen der allgemeinen Formel (IIj)

   R^{53'}-A-D-E-G-L-R² (IIj)

   in welcher
   - R^{53'}: für 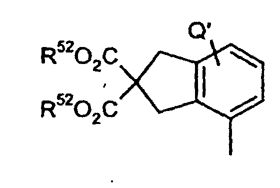 steht, worin
   Q', R⁵² die oben genannte Bedeutung haben,
   - A, D, E, G, L und R²: die oben genannte Bedeutung haben,
   reduziert werden,
   oder
[H] Die Verbindungen der allgemeinen Formel (IIk) 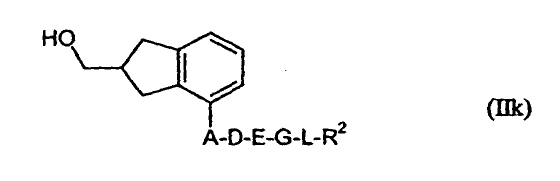 in welcher
   - A, D, E, G, L und R²: die oben angegebene Bedeutung haben,
   können durch ein neues Verfahren hergestellt werden, dadurch gekennzeichnet, daß man die Verbindungen der allgemeinen Formel (IIz) 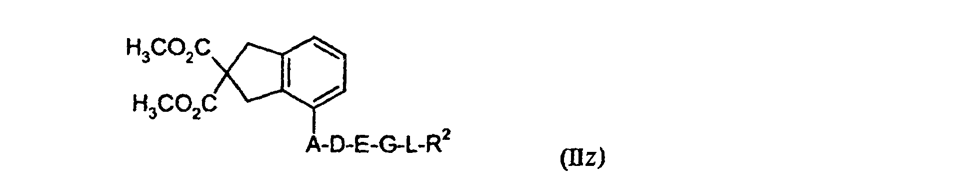 in welcher
   - A, D, E, G, L und R2: die oben angegebene Bedeutung haben,
   durch Einsatz von HBr und Essigsäure in die Verbindungen der allgemeinen Formel (IIm) 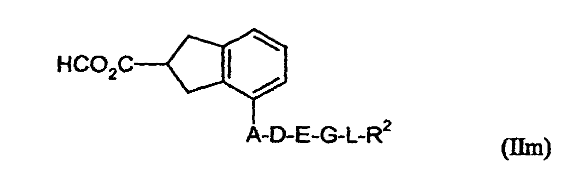 in welcher
   - A, D, E, G, L und R2: die oben angegebene Bedeutung haben,
   überführt,
   und in einem letzten Schritt eine Reduktion mit BH₃ x S(CH₃)₂ in Tetrahydrofuran durchführt,
   und im Fall der reinen Enantiomeren eine HPLC-Trennung nach üblichen Methoden durchführt,
   und gegebenenfalls die oben aufgeführten Substituenten nach üblichen Methoden eingeführt und derivatisiert werden,
   und im Fall D ist = -SO- oder -SO₂- ausgehend von den entsprechenden Thioethern (D = S) eine Oxidation nach üblichen Methoden durchgeführt wird,
   und im Fall der Ammoniumverbindungen ausgehend von den entsprechenden Aminen eine Alkylierung durchgeführt wird.

Die erfindungsgemäßen Verfahren können durch folgende Formelschemata beispielhaft erläutert werden:

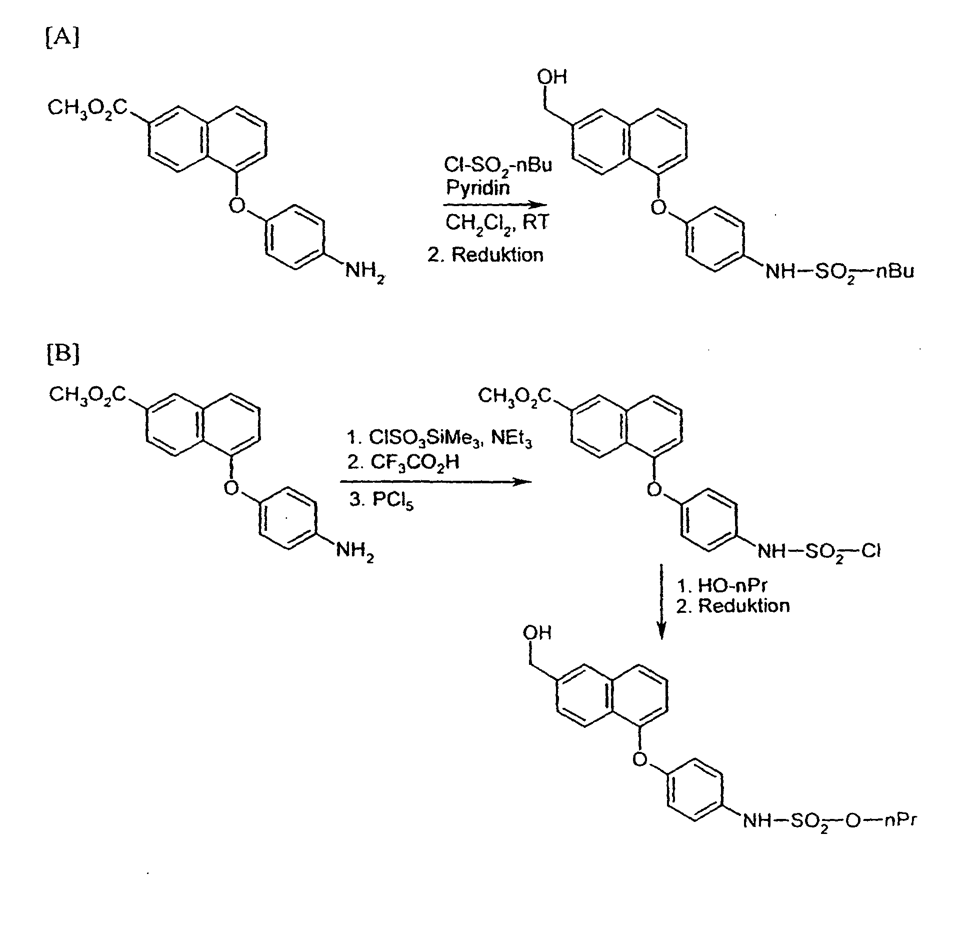

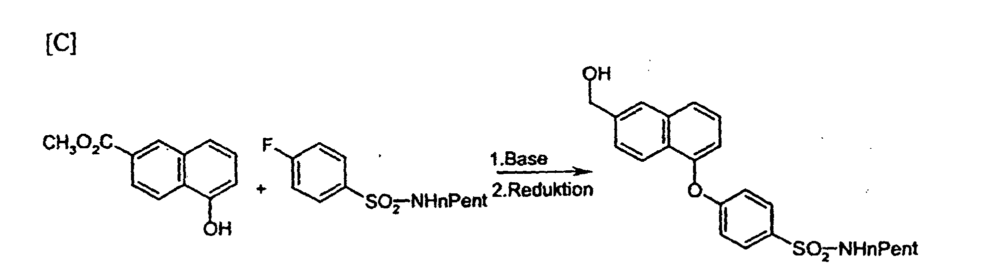

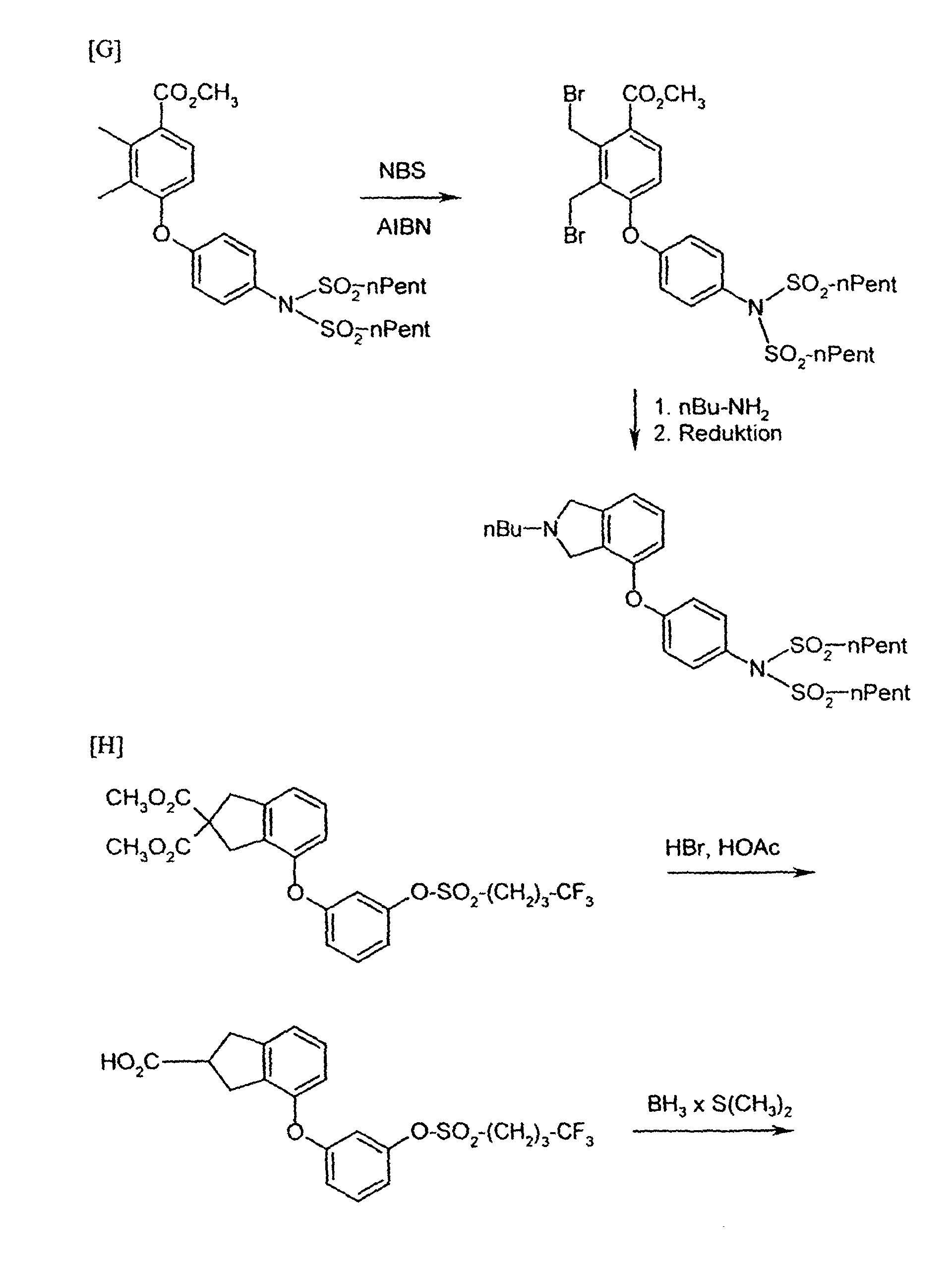

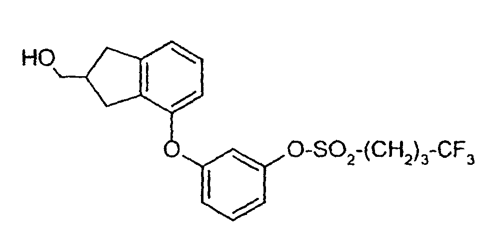

Als Lösemittel eignen sich Ether wie Diethylether, Dioxan, Tetrahydrofuran, Glykoldimethylether, oder Kohlenwasserstoffe wie Benzol, Toluol, Xylol, Hexan, Cylcohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dichlormethan.

Als Basen eignen sich im allgemeine Alkalihydride oder -alkoholate, wie beispielsweise Natriumhydrid oder Kalium-tert.butylat, oder cyclische Amine, wie beispielsweise Piperidin, Pyridin, Dimethylaminopyridin oder C₁-C₄-Alkylamine, wie beispielsweise Triethylamin. Bevorzugt sind Triethylamin, Natriumhydrid, Pyridin und/oder Dimethylaminopyridin.

Als Basen eignen sich außerdem üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kaliumtert.butanolat. Besonders bevorzugt sind Kaliumcarbonat und Natriumhydroxid.

In einer Variante wird die Umsetzung in Pyridin, dem eine katalytische Menge DMAP zugesetzt wird, durchgeführt. Gegebenenfalls kann noch Toluol zugefügt werden.

Die Verfahren werden im allgemeinen bei Normaldruck durchgeführt. Es ist aber auch möglich, die Verfahren bei Überdruck oder bei Unterdruck durchzuführen (z.B. in einem Bereich von 0,5 bis 5 bar).

Die Verbindungen der allgemeinen Formel (IIh) sind teilweise bekannt oder neu und können durch Umsetzung der Verbindungen der allgemeinen Formeln (XIII) und (XIV) 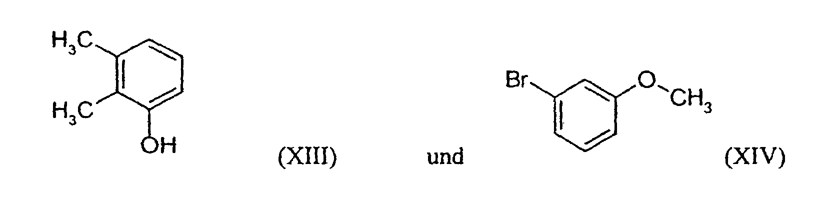 in Anwesenheit von CuO (kat.), Kaliumcarbonat und Pyridin, die Verbindungen der allgemeinen Formel (XV) 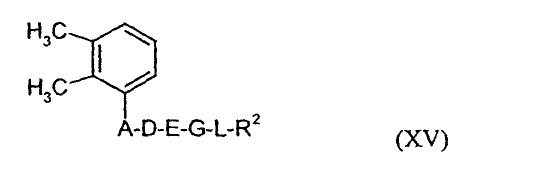 in welcher
- A, D, E, G, L und R²: die oben angegebene Bedeutung haben,
herstellt,
und abschließend mit Bromwasserstoffsäure und Eisessig die Hydroxyfunktion freisetzt.

In DOS 1 942 264 wird die Herstellung von fluorierten Alkansulfonsäurechloriden beschrieben, in US 5 149 357 u.a. die Herstellung eines 4,4,4-Trifluorbutansulfonsäureamids, ohne jedoch die Herstellung des entsprechenden Sulfonsäurechlorids zu offenbaren.

Die fluorierten Sulfonsäurechloride wurden analog DOS 1 942 264 hergestellt.

Die Verbindungen der allgemeinen Formeln (V), (VII), (X), (XI), (XII), (XIII) und (XIV) sind an sich bekannt oder nach üblichen Methoden herstellbar.

Die Verbindungen der allgemeinen Formeln (IIa), (V'), (VI), (VII'), (IIb), (VIII), (IX), (IX'), (IIc), (IId), (IIe), (IIf), (X'), (IIi), (IIj), (IIk), (IIl), (IIm) und (XV)können wie oben beschrieben hergestellt werden.

Die Alkylierung zur Herstellung der Ammoniumverbindungen erfolgt im allgemeinen mit Alkylierungsmitteln wie beispielsweise Alkylhalogenide, Sulfonsäureester oder substituierte oder unsubstituierte Dialkyl- oder Diarylsulfonate, vorzugsweise mit Methyljodid oder Dimethylsulfat.

Die Alkylierung erfolgt im allgemeinen in einem der oben aufgeführten Lösemitteln, vorzugweise in Dimethylformamid in einem Temperaturbereich von 0°C bis +70°C, vorzugsweise von 0°C bis +30°C und Normaldruck.

Bevorzugt sind Verbindungen der allgemeinen Formel (I), deren Löslichkeit in 0,9%iger wäßriger Kochsalzlösung bei 25°C bei mehr als 10mg/l, besonders bevorzugt bei mehr als 100 mg/l liegt.

Außerdem sind solche Aminosäureester der allgemeinen Formel (I) bevorzugt, in *in vivo* zum entsprechenden Alkohol der allgemeinen Formel (II) hydrolysiert werden.

Überraschenderweise zeigen die neuen Aminosäureester von Arylsulfonamiden und ihren Analoga ein nicht vorhersehbares, wertvolles pharmakologisches Wirkspektrum.

Sie zeichnen sich als hochwirksame Agonisten des CB1-Rezeptors und teilweise des CB2-Rezeptors aus. Sie können allein oder in Kombination mit anderen Arzneimitteln eingesetzt werden zur Behandlung und/oder Prävention von neuronalen Schädigungen unterschiedlicher Ursache wie beispielsweise durch ischämischen, thromb- und/oder thrombemolischen, und hämorrhagischen Schlaganfall, Zuständen nach direkten und indirekten Verletzungen im Bereich des Gehirnes und des Schädels. Ferner zur Behandlung und/oder Prävention von cerebralen Ischämien nach sämtlichen operativen Eingriffen am Gehirn oder peripheren Organen bzw. Körperteilen und damit einhergehenden oder vorausgehenden Zuständen krankhafter bzw. allergischer Natur, die primär und/oder sekundär zu einer neuronalen Schädigung führen können. Gleichfalls eignen sich die erfindungsgemäßen Verbindungen auch zur Therapie von primären und/oder sekundären krankhaften Zuständen des Gehirnes, beispielsweise während oder nach cerebralen Vasospasmen, Migräne, Spastizität Hypoxie und/oder Anoxie nicht vorher genannter Genese, perinataler Asphyxie, Autoimmunerkrankungen, Stoffwechsel- und Organerkrankungen, die mit einer Schädigung des Gehirnes einhergehen können sowie Schädigungen des Gehirnes infolge primärer Gehirnerkrankungen beispielsweise Krampfleiden und arteround/oder arteriosklerotischer Veränderungen. Zur Behandlung chronischer oder psychiatrischer Leiden wie beispielsweise Depression neurodegenerativer Erkrankungen wie beispielsweise Alzheimersche, Parkinsonsche oder Huntingtonsche Erkrankung, Multiple Sklerose, amyotrophische laterale Sklerose, Neurodegeneration durch akute und/oder chronische virale oder bakterielle Infektionen und Multiinfarktdemenz.

Darüber hinaus können sie in Arzneimitteln eingesetzt werden zur Behandlung von Schmerzzuständen, Emesis, Übelkeit, Glaukom, Asthma, Anorexie, Konvulsionen, Rheuma, Sedation und Bewegungsstörungen.

Die erfindungsgemäßen Substanzen eignen sich auch zur Behandlung von Erkrankungen, die durch bakterielle und/oder virale Infektion verursacht werden, die auf direkte und/oder indirekte Veränderungen des Immunsystems bzw. auf Fehlsteuerungen unter Mitwirkung des Immunsystems beruhen, wie z.B. bei lokalen oder systemischen Autoimmunerkrankungen (z.B. Lupus erythematodes in allen seinen Varianten), entzündlichen und/oder autoimmunologisch bedingten Erkrankungen der Gelenke (z.B. primär chronische Polyarthritis, traumatisch bedingten Entzündungen), entzündlichen und/oder autoimmunologisch bedingten Erkrankungen des Knochen- und Muskelapparates, entzündlichen und/oder autoimmunologisch bedingten krankhaften Prozessen der inneren Organe (z.B. Morbus Crohn, Glomerulonephritis) und der äußeren Organe (z.B. allergische Reaktionen durch aerogene Aufnahme von Antigenen) und des zentralen Nervensystems (z.B. Multiple Sklerose, Morbus Alzheimer, psychiatrische Erkrankungen) sowie der Sinnesorgane, primären und/oder sekundären und/oder autoimmunologischen Erkrankungen des blutbildenden Systems und des Immunsystems (z.B. Abstoßungsreaktionen, AIDS) selbst, sowie bei Hauterkrankungen entzündlicher und/oder immunologischer Genese bei Mensch und Tier. Ferner wirken diese Substanzen bei den indirekten Symptomen dieser Erkrankungen wie z.B. Schmerz.

Bevorzugt ist ihre Verwendung zur Behandlung von Schmerz, Spastizität, cerebralen Ischämien und Schädel/Hirn-Trauma.

Zur Löslichkeitsbestimmung wurde eine Fällungsmethode herangezogen:

10 mg der Testsubstanz werden in 50µl DMSO vollständig gelöst (Stammlösung). Von dieser Lösung gibt man 20µl in 2000µl physiologische Kochsalzlösung. Diese Lösung wiederum wird zur Equilibrierung bei 25°C im Thermomixer Comfort (Fa. Eppendorf) bei 1400 rpm 1 Stunde geschüttelt.
Die ausgefallenen Teile der Testsubstanz werden mit der Biofuge 15 Fa. Heraeus 5 min bei 14000 rpm abzentrifugiert. 1300 µl des Überstandes werden erneut mit der Microfuge Fa. Beckmann bei 45000 rpm = 125000g zentrifugiert.
10 µl dieses Zentrifugationsüberstandes werden nun mit 1000µl DMSO verdünnt und diese Lösung an der HPLC gemessen. (FA. Hewlett Packard 1090, Methode: Gradient von 100% PBS-Puffer pH=4 innerhalb von 15 min auf 10% Puffer/90% Acetonitril, Säule: RP18)
Die gemessene Peakfläche der HPLC-Messung wird mit einer Eichgerade auf die Substanzkonzentration umgerechnet. Für die Eichgerade werden 20µl der Stammlösung sukzessiv mit DMSO so verdünnt, daß 5 Konzentrationen von 2.5 mg/l bis 2000mg/l entstehen. Diese Lösungen werden ebenfalls an der HPLC gemessen (Methode s. o.) und die Peakflächen gegen die Konzentrationen aufgetragen.

Nach dieser Löslichkeitsbestimmungsmethode hatte das Beispiel 5 eine Löslichkeit von 720 mg/l.

### CB1-Luciferase Reportergen Test

### 1. Klonierung des Ratten Cannabinoid Rezeptors CB1

Gesamt-RNA aus Ratten-Hirn (das Gewebe wurde frisch getöteten Tieren entnommen und in flüssigem Stickstoff schockgefroren) wurde durch saure Guanidinium-Thiocyanat/Phenol/Chloroform-Extraktion (J. Biol. Chem. 1979, 18, 5294) isoliert und mittels reverser Transkriptase und Random-Primem (jeweils von Invitrogen) in cDNA überführt. Die Polymerase Ketten Reaktion (PCR, Bedingungen: 4 min 94°C, 1x; 1 min 94°C; 2 min 53°C; 1 min 72°C, 50 Zyklen; 1 min 94°C, 2 min 53°C, 4 min 72°C, 1x) wurde in einem Perkin Elmer Thermocycler mit dem Enzym Taq Polymerase (Perkin Elmer) durchgeführt; die eingesetzten Oligonukleotid-Primer (Basen 99 bis 122: 5'→3', "down"; 1556-1575: 3'←5', "up") waren von der publizierten Sequenz des Ratten Cannabinoid- Rezeptors (Nature 1990, 346, 561) abgeleitet und wurden auf einem DNA Synthesizer, Modell 1380 der Fa. Applied Biosystems. synthetisiert. Ein Teil der PCR-Reaktion wurde in einem 1 %igen Agarose-Gel in 1x TBE-Puffer aufgetrennt und anschließend mit Ethidium-Bromid angefärbt, wobei nur eine Bande mit der erwarteten Länge sichtbar war (etwa 1,5 kb). Dieses PCR-Produkt wurde in den TA-Cloning Vektor (Invitrogen) subkloniert und die Nukleotid-Sequenz des Inserts mit T7DNA Polymerase (Sequenase, USA/Amersham) durch die Dideoxynukleotid-Kettenabbruch-Reaktion bestimmt. Das Insert besitzt eine Länge von 1477 Basenpaaren und enthält ein offenes Leseraster von 1419 Basenpaaren was einem Protein von 473 Aminosäuren entspricht. Die Anzahl der Basenpaare, die Position des offenen Leserasters und die Anzahl der Aminosäuren stimmen mit der publizierten Sequenz überein. Computer-Analysen wurden mit Hilfe der GCG Software Suite (Genetic Computer Group) durchgeführt. Das cDNA Insert wurde nach Partialverdauung mit HindIII und Notl (Biolabs) in den Expressionsvektor pRc/CMV (Invitrogen) subkloniert. Dieses Konstrukt (Plasmid CMV-RH) wurde für Transfektions-Experimente eingesetzt.

### 2. Stabile Transfektion der CHOluc9 Reporter Zellen

CHOluc9 Zellen wurden in 50 % Dulbecco's modifiziertem Eagle Medium / 50 % F-12 (DMEM/F12) gezüchtet, das 10 % foetales Kälberserum (FCS) enthielt. Transfektionen wurden in 6-well Platten angesetzt. 7,5 µg Qiagen-gereinigte CMV-RH Plasmid DNA wurde pro 105 Zellen mit dem DOTAP Transfektions System zugegeben, entsprechend dem Versuchsprotokoll des Herstellers (Boehringer Mannheim). Transfizierte Zellen wurden mit 1 mg/ml G418 selektioniert und Einzelklone wurden durch Limiting Dilution auf 96-well Platten erhalten. Zell-linien, die den Cannabinoid-Rezeptor exprimieren, wurden nach Inkubation mit dem Cannabinoid-Rezeptor Agonisten, WIN-55,212-2, in Gegenwart von Forskolin an der Hemmung der Reportergen-Expression identifiziert. Mehrere stabil transfizierte und subklonierte Zellinien wurden mittels RT-PCR, wie unter I. beschrieben, weiter charakterisiert.

### 3. Test-Optimierung und pharmakologische Charakterisierung der CHOCB1 Reporter-Zellinie

Der Luciferase-Test wurde mit dem Ziel hoher Sensitivität und Reproduzierbarkeit, geringer Varianz und guter Eignung für die Durchführung auf dem Robotersystem optimiert durch Variation mehrerer Testparameter, wie z.B. Zelldichte, Dauer der Anzuchtphase und der Testinkubation, Forskolin-Konzentration, Medium-Zusammensetzung. Zur pharmakologischen Charakterisierung der Zellen und zum Robotergestützten Substanz-Screening wurde das folgende Testprotokoll verwendet: Die Stammkulturen wurden in 50 % Dulbecco's modifiziertem Eagle Medium / 50 % F-12 (DMEM/F12) mit 10 % FCS bei 37°C unter 10 % CO₂ gezüchtet und jeweils nach 2 bis 3 Tagen 1:10 gesplittet. Testkulturen wurden mit 5000 Zellen pro Napf in 96-well Platten ausgesät und 70 Stunden bei 37°C angezogen. Dann wurden die Kulturen vorsichtig mit Phosphat-gepufferter Saline gewaschen und mit serumfreiem Ultra-CHO Medium (Bio-Whittaker) rekonstituiert. Die in DMSO gelösten Substanzen wurden 1 x in Medium verdünnt und zu den Testkulturen pipettiert (maximale DMSO-Endkonzentration im Testansatz: 0,5 %). 20 Minuten später wurde Forskolin zugegeben und die Kulturen anschließend 3 Stunden im Brutschrank bei 37°C inkubiert. Danach wurden die Überstände entfernt und die Zellen durch Zugabe von 25 µl Lysereagens (25 mM Triphosphat, pH 7,8 mit 2mM DTT, 10 % Glycerin, 3 % TritonX100) lysiert. Direkt danach wurde Luciferase Substrat Lösung (2,5mM ATP, 0,5 mM Luciferin, 0,1 mM Coenzym A, 10mM Tricin, 1,35mM MgSO4, 15mM DTT, pH 7,8) zugegeben, kurz geschüttelt, und die Luciferase-Aktivität mit einem Hamamatzu Kamerasystem gemessen.

Zur Inaktivierung von Gᵢ-Proteinen wurden die Testkulturen vor dem Test für 16 Stunden mit 5 ng/ml (Endkonz.) Pertussis Toxin behandelt.

Die IC₅₀-Werte wurden mit dem Programm GraphPadPrism berechnet (Hill-Gleichung, speziell: one-site competition).

### Aktivität im Ratten CB 1-Rezeptor-Luciferase Rezeptorgen Test

| **Beispiel** | **IC**_{**50**} **(nmol/l)** |
|---|---|
| 1 | 0,35 |
| 2 | 0,13 |
| 5 | 0,11 |
| 6 | 0,85 |
| 7 | 0,4 |
| 8 | 0,2 |

### hCB2-Luciferase Reportergen Test

CHOluc9 Zellen wurden mit dem humanen CB2-Rezeptor stabil transfiziert. Transfektion, Klonselektion und Testentwicklung wurden analog zu den Arbeiten mit dem Ratten CB1-Rezeptor durchgeführt. Das folgende Testprotokoll wurde zur pharmakologischen Charakterisierung der Zellen und zur Substanz-Testung verwendet:

Die Starnmkulturen wurden in 50% Dulbecco's modifizierten Eagle Medium/50% F-12 (DMEM/F12) mit 10% FCS bei 37°C unter 10% CO₂ gezüchtet und jeweils nach 2 bis 3 Tagen 1:10 gesplittet. Testkulturen wurden mit 5000 Zellen pro Napf in 96-well-Platten in DMEM/F12 Medium mit 5 % FCS ausgesät und 70 Stunden bei 37°C angezogen. Dann wurde das Medium von den Kulturen entfernt und durch serumfreies Ultra-CHO Medium (Bio-Whittaker) ersetzt. Die in DMSO gelösten Substanzen (200x Endkonzentration) wurden zu den Testkulturen pipettiert (maximale DMSO-Endkonz. im Testansatz: 0,5%) und 20 min später wurde Forskolin zugegeben. Anschließend wurden die Kulturen 3,5 Stunden im Brutschrank bei 37°C inkubiert. Danach wurden die Überstände entfernt und die Zellen durch Zugabe von 25 µl Lysereagens (25 mM Trisphosphat, pH 7,8 mit 2 mM DTT, 10 % Glycerin, 3 % Triton X 100) lysiert. Direkt anschließend wurden 50 µl Luciferase Substrat Lösung, doppelt konzentriert, (5 mM ATP, 1 mM, Luciferin, 0,2 mM Coenzym A, 10 mM Tricin, 1,35 mM MgSO₄, 15 mM DTT, pH 7,8) zugegeben, kurz geschüttelt, und die Luciferase-Aktivität mit einem Photomultiplier-Kamera-Meßsystem (Hamamatzu) bestimmt.

Die IC₅₀-Werte wurden mit dem Program GraphPad Prism™ berechnet (Hill-Gleichung; speziell: one site competition).

### Bindungsstudien an Ratten Cortex Membranen

Membranprotein wird nach Standardmethoden aus unterschiedlichen Geweben bzw. von Zellen präpariert. Puffer, markierter Ligand, DMSO oder Teststubstanz werden zusammenpipettiert, anschließend werden 100 µg Protein hinzugegeben, die Mischung gut vermischt und 60 min bei 30°C im Wasserbad inkubiert. Nach Ablauf der Inkubationszeit wird die Reaktion durch Zugabe von eiskaltem Inkubationspuffer in jedes Röhrchen gestoppt. Nach Abfiltrieren wird mit 3/4 ml Inkubationspuffer nachgewaschen. Die Filter werden in Minivials überführt, die Radioaktivität wird in einem Flüssigszintillationszähler bestimmt.

### Inhibition der Glutamat-Freisetzung

Nach Dekapitieren einer Ratte wird der Schädel eröffnet, das Gehirn herausgehoben und entlang der Mittelfurche durchschnitten. Der Hippocampus wird freipräpariert, vom restlichen Gewebe getrennt, in 350 µM dicke Schnitte geschnitten und für 60 min in Siebgefäßen bei 37°C inkubiert. Gefolgt von Basalwert und Stimulation 1 mit 75 mM KCl (S1) werden die Schnitte mit Testsubstanz inkubiert und dann die Stimulation mit KCl und Testsubstanz (S2) wiederholt. Die Glutamat-Konzentration der zu untersuchenden Proben wird dann über eine enzymatische Reaktion (GLDH) und fluorometrischer Messung von NADH gemessen. Anhand einer Eichkurve wird der Glutamatgehalt der Probe bestimmt, und unter Kenntnis des Proteingehaltes kann der Glutamatgehalt/mg Protein errechnet werden. Verglichen wird das Verhältnis S2/S1, Glutamat-Freisetzungsinhibitoren reduzieren dieses Verhältnis konzentrationsabhängig.

Mit der folgenden Testmethode kann die *in vitro*-Umwandlung der erfindungsgemäßen Aminosäureester in die entsprechenden Alkohole bestimmt werden.

### Bestimmung der Stabilität von Substanzen im Blut verschiedener Spezies (Ratte, Hund, Human)

### Prinzip der Methode

Die Testsubstanz wird in heparinisiertem Blut jeder Testspezies inkubiert. Zu geeigneten Zeitpunkten werden Aliquote des Ansatzes entnommen und in eine Acetonitrilvorlage pipettiert. Nach Zentrifugation wird der Überstand eingedampft und der Rückstand in einem für die Analytik geeigneten Lösungsmittel aufgenommen.

| **Material** | |
|---|---|
| Laborzentrifuge: | Sigma 4K10 |
| | (Sigma Laborzentrifugen, Osterode, Germany) |
| Schüttler: | KS500 |
| | (Janke und Kunkel, IKA Labortechnik, Staufen, |
| | Germany) |
| Wasserbad, Thermomix® | 1442D (Braun-Melsungen, Melsungen, |
| | Germany) |
| Abdampfvorrichtung | BAYER AG |

### Durchführung

Zur Bestimmung der Stabilität einer Testsubstanz *in vitro* wird die Substanz, die in einem kleinen Volumen eines geeigneten Lösungsmittels gelöst ist, in einer Konzentration von z.B.
2 µg/ml in 5 ml Blut bei 37 °C über 5 Stunden inkubiert. Zu geeigneten Zeitpunkten werden100 µl des Ansates zu 500 µl Acetonitrilvorlage pipettiert und gemischt. Nach Zentrifugation bei 3000 rpm wird der Überstand entnommen und in einem Wasserbad bei
40 °C zur Trockne eingedampt. Der Rückstand wird in einem für die Analytik geeigneten Lösungsmittel aufgenommen.

| | |
|---|---|
| Lösungsmittel | 10 µl EtOH / 5 ml Blut |
| Schüttlergeschwindigkeit | 250 rpm |
| Zentrifugation | 3000 rpm |
| Zentrifugationszeit | 10 min |
| Blutvolumen | 5 ml |
| Blutaliquote | 100 µl |
| Inkubationszeiten | 0, 2, 5, 10, 15, 30, 45 Minuten, 1, 2, 3, 5 Stunden |

Mit den folgenden Testmethoden können die in vivo-Umwandlung der erfindungsgemäßen Aminosäureester in die entsprechenden Alkohole bestimmt werden.

### Pharmakokinetik der Substanzen in der Ratte

### 1. Intravenöse Infusion

Die Substanz wird über einen Venenkatheter (Introcan®, 22G1, Braun, Melsungen, Germany) über eine laterale Schwanzvene direkt in den Blutstrom infundiert. Für die exakte Verabreichung der gewählten Dosis und des Volumens wird eine kalibrierte 10 ml Spritze verwendet. Für die Infusion wird die Pumpe Nr.540210 von TSE, Bad Homburg, FRG benutzt.

### 2. Probennahme und Aufarbeitung

### Blut und Plasma

Blutproben werden von katheterisierten Tieren (Vena jugularis) in heparinisierten Röhrchen gesammelt. Das Blut wird zentrifugiert und das Plasma auf geeignete Weise für die Analytik vorbereitet. Das Plasma wird bis zur Analytik bei < -15°C aufbewahrt.

### Pharmakokinetik der Substanzen im Hund

### 1. Intravenöse Infusion

Nach Kannülierung einer oberflächlichen Vene am Vorder- oder Hinterlauf wird die Substanz direkt in den Blutstrom infundiert. Der Venenkatheter (z.B. Introcan® 20 G / 1¼, B. Braun, Melsungen, Germany) wird mit einer kalibrierten Spritze, welche an der Infusionspumpe befestigt ist, verbunden.

### 2. Probennahme und Aufarbeitung

### Blut und Plasma

Blutproben werden durch Punktion einer oberflächlichen Vene am Vorder- oder Hinterlauf oder einer Jugularvene entnommen. Die für die Infusion benutzte Extremität wird für die Blutentnahme nicht verwendet. Das Blut wird zentrifugiert und das Plasma bis zur Analytik bei <-15 °C aufbewahrt.

### Hypothermie

### 1. Agonismus Prüfung:

Fünf Minuten nach Bestimmung der Basal-Körpertemperatur via Oesophagus Temperatursonde wird die Prüfsubstanz (i.v.) appliziert. Eine Kontrollgruppe erhält, ebenfalls i.v., nur das Lösungsmittel der Prüfsubstanzen. Die Körpertemperatur wird 7,5, 15, 30 und 60 Minuten nach i.v.-Applikation gemessen. Die Gruppengröße pro Dosis beträgt 5-7 Tiere (Ratten).

### 2. Antagonismus Prüfung:

60 Minuten vor Prüfsubstanz Applikation wird der spezifische CB1 Antagonist *SR 141716A*, der Kontrollgruppe nur das Lösemittel (Solutol/0,9% NaCl) intraperitoneal appliziert. Die basale Körpertemperatur wird fünf Minuten vor Applikation von *SR 141716A* via Oesophagus Temperatursonde gemessen. Das weitere Vorgehen entspricht der Methode "Agonismus Prüfung". Die Gruppengröße pro Dosis beträgt 5-7 Tiere (Ratten).

### Ratten Hypothermie - Agonismus Prüfung

| **Beispiel** | **ED**_{**-1°C**} ^{**a)**}**[mg/kg]** |
|---|---|
| 5 | 0,03 |

| | |
|---|---|
| a) Effektive Dosis für 1°C Körpertemperatur-Reduktion b) Die Hypothermie wird durch Applikation des spezifischen CB1-Antagonisten SR 141716 A signifikant reduziert (siehe Methode "Antagonismus Prüfung") | |

### Permanente focale cerebrale Ischämie bei der Ratte (MCA-O)

Unter Isofluran Anästhesie wird die Arteria cerebri media einseitig freipräpariert mittels Elektrokoagulation diese und deren Nebenäste irreversibel verschlossen. Als Folge des Eingriffs entsteht ein cerebraler Infarkt. Während der Operation wird die Körpertemperatur des Tieres auf 37°C gehalten. Nach Wundverschluß und Abklingen der Narkose werden die Tiere wieder in ihren Käfig entlassen. Die Substanzapplikation erfolgt nach unterschiedlichen zeitlichen Schemata und über unterschiedliche Applikationswege (i.v, i.p.) nach der Okklusion. Die Infarktgröße wird nach 7 Tagen bestimmt. Dazu wird das Gehirn entnommen, histologisch aufgearbeitet und mit Hilfe eines computergestützten Auswertsystemes das Infarktvolumen bestimmt.

### Subdurales Hämaton bei der Ratte (SDH)

Unter Anästhesie wird den Tieren einseitig subdural Eigenblut injiziert. Unter dem Hämatom bildet sich ein Infarkt. Die Substanzapplikation erfolgt nach unterschiedlichen zeitlichen Schemata und über unterschiedliche Applikationswege (i.v., i.p.). Die Bestimmung der Infarktgröße erfolgt wie beim Modell der Permanenten focalen Ischämie bei der Ratte (MCA-O) beschrieben.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, insbesondere perlingual oder intravenös.

Im allgemeinen hat es sich als vorteilhaft erwiesen, bei intravenöser Applikation Mengen von etwa 0,01 bis 10 mg/kg, vorzugsweise etwa 0,1 bis 10 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Ausgangsverbindungen

### Beispiel 1A

### Thiocyansäure-4,4,4-trifluorbutylester

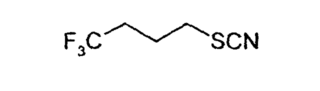

Eine gerührte Lösung von 4,4,4-Trifluorbutanol (35 g; 0,027 mol) und Triethylamin (28,3 g; 0,280 mol) in 200 ml Dichlormethan wurde bei 0°C tropfenweise mit einer Lösung von Methansulfonsäurechlorid (32,1 g; 0,280 mol) in 100 ml Dichlormethan versetzt. Nach Ende der Zugabe wurde weitere 30 min gerührt, dann auf Eis gegossen und anschließend die Phasen getrennt. Die organische Phase wurde über Magnesiumsulfat getrocknet und unter vermindertem Druck aufkonzentriert. Es wurden 55 g rohes 4,4,4-Trifluorbutyl-methansulfonat als oranges Öl erhalten.

Das Mesylat (55 g) wurde mit Natriumthiocyanat (30,6 g; 0,30 mol) in Aceton (300 ml) 6 h unter Rückfluß gekocht. Nach Abkühlung auf Raumtemperatur wurde die Mischung auf Eis gegossen, die Phasen getrennt und die organische über Magnesiumsulfat getrocknet. Nach Filtration und Aufkonzentrieren unter vermindertem Druck wurden 41 g (89 % d.Th.) Thiocyansäure-4,4,4-trifluorbutylester als Öl erhalten.
¹⁹F-NMR (376 MHz, CDCl₃; CFCl₃) δ [ppm]: -66,3
¹H-NMR (400 MHz, CDCl₃, TMS) δ [ppm]: 2,15 (m, 2H); 2,3 (m, 2H); 3,05 (t, J = 7,1 Hz, 2H)

### Beispiel 2A

### 4,4,4-Trifluorbutansulfonsäurechlorid

F₃C-CH₂-CH₂-CH₂-SO₂Cl

In eine Lösung von Beispiel 1 A (40 g; 0,236 mol) in wäßriger Essigsäure (150 ml Essigsäure und 70 ml Wasser) wurde bei 20 bis 40°C Chlor eingeleitet und der Fortschritt der Reaktion gaschromatographisch verfolgt. Als die Chlorierung vollständig war, wurde der Überschuß Chlor mittels Durchleitung eines Stickstoffstromes verdrängt, 200 ml Wasser zugefügt und die Reaktionsmischung mit Dichlormethan mehrfach extrahiert. Die vereinigten organischen Phasen wurden über Magnesiumsulfat getrocknet, davon abfiltriert und unter vermindertem Druck aufkonzentriert. Man erhielt 44 g (89 % d.Th.) 4,4,4-Trifluorbutansulfonsäurechlorid als gelbes Öl.
¹⁹F-NMR (376 MHz, CDCl₃; CFCl₃) δ [ppm]: -66,65 (t, J = 10 Hz)
¹H-NMR (400 MHz, CDCl₃, TMS) δ [ppm]: 3,8 (m, 2H); 2,35 (m, 4H)

### Beispiel 3A

### 3-(2,3-Dimethylphenyloxy)-anisol

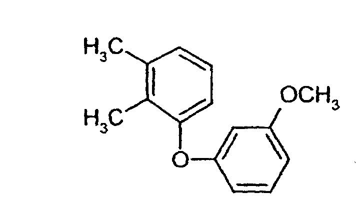

Eine Lösung von 2,3-Dimethylphenol (341,0 g; 2,79 mol) und 3-Bromanisol (548,2 g; 2,93 mol) in Pyridin (3000 ml) wird mit K₂CO₃ (771,5 g; 5,58 mol) und Kupfer-(II)-oxid (44,4 g; 0,56 mol) versetzt und unter Argon 36 h unter Rückfluß gerührt. Nach Zugabe von Kupfer-(II)-oxid (20 g; 0,25 mol) wird weitere 24 h unter Rückfluß gerührt. Der Ansatz wird nach dem Abkühlen filtriert, der Rückstand mit Dichlormethan gewaschen und das Filtrat wird i.V. eingeengt. Der Rückstand wird in Diethylether (3000 ml) aufgenommen und mit Wasser (300 ml) gewaschen. Ausgefallener Feststoff wird abgesaugt und nach Phasentrennung wird die organische Phase mit 2 N HCl (3 x 300 ml), Wasser (300 ml), 10% Natronlauge (3 x 300 ml) und Wasser (300 ml) gewaschen. Die Etherphase wird getrocknet (MgSO₄) und i.V. eingeengt. Der Rückstand wird i.V. destilliert.
Ausbeute: 441,5 g (68% d.Th.)
Sdp.: 112°C / 0,1 mbar
MS (DCI, NH₃): m/z = 246 (M+NH₄)

### Beispiel 4A

### 3-(2,3-Dimethylphenyloxy)-phenol

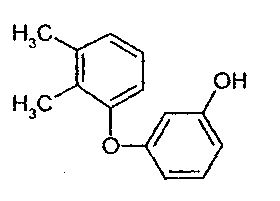

Beispiel 3A (109,6 g; 480 mmol) wird in 48 % wäßrigem Bromwasserstoff (900 ml) und Essigsäure (1500 ml) vorgelegt und über Nacht unter Rückfluß gerührt. Anschließend wird der Ansatz im Vakuum eingeengt, der Rückstand in Wasser aufgenommen und dreimal mit Ethylacetat extrahiert. Die vereinten organischen Phasen werden zweimal mit Wasser gewaschen, getrocknet (MgSO₄) und im Vakuum eingeengt. Der Rückstand wird an Kieselgel mit Toluol:EE (10:1) chromatographiert.
Ausbeute: 86,5 g (83 % d.Th.)
R_{f} = 0,15 (Toluol)
MS (ESI): m/z = 215 (M+H)

### Beispiel 5A

### 4,4,4-Trifluor-1-butansulfonsäure-3-(2,3-dimethyl-phenyloxy)-phenylester

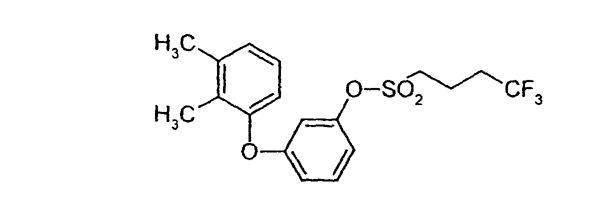

Die Herstellung erfolgt in Analogie zur Herstellung des Beispiels 1 ausgehend von Beispiel 4A (4,54 g; 21,2 mmol).
Ausbeute: 7,80 g (95% d.Th.)
R_{f} = 0,51 (Toluol)
MS (DCI / NH₃): m/z = 406 (M+NH₄)

### Beispiel 6A

### 4,4,4-Trifluormethyl-1-butansulfonsäure-3-(2,3-bis-brommethylphenyloxy)-phenylester

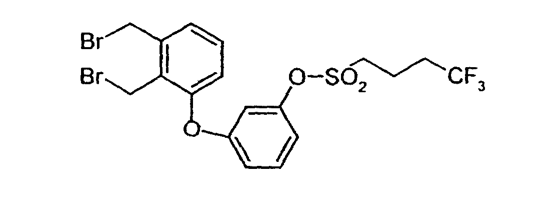

Eine Lösung von Beisiel 5A (6,76 g; 17,4 mmol) in Tetrachlorkohlenstoff (150 ml) wird mit N-Bromsuccinimid (6,50 g; 36,5 mmol) versetzt, auf Rückfluß erhitzt und unter Rühren 5 h mit einer 300 W-Lampe bestrahlt. Nach dem Abkühlen wird ausgefallenes Succinimid abgesaugt und das Filtrat im Vakuum eingeengt. Der Rückstand wird an Kieselgel mit Toluol chromatographiert. Man erhält ein Gemisch (ca. 5:1) von Beispiel 6A und 4,4,4-Trifluor-1-butansulfonsäure-3-(2-Brommethyl-3-dibrommethylphenyloxy)-phenylester (9,9 g), das ohne weitere Reinigung weiter verwendet wurde.

### Beispiel 7A

### 4,4,4-Trifluor-1-butansulfonsäure-3-(2,2-bis-methoxycarbonyl-indanyl-4-oxy)-phenylester

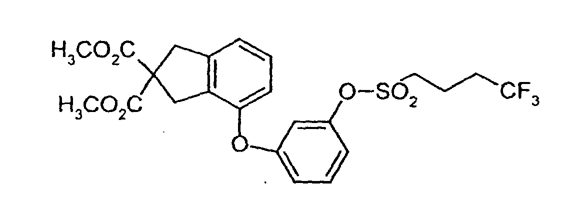

Das in Beispiel 6A erhaltene ca. 5:1-Gemisch von 4,4,4-Trifluor-1-butan-sulfonsäure 3-(2,3-bis-brommethylphenyloxy)-phenylester und 4,4,4-Trifluor-1-butansulfonsäure-3-(2-Brommethyl-3-dibrommethylphenyloxy)-phenylester (6,00 g) wird in 2-Butanon (150 ml) gelöst. Nach Zugabe von Malonsäuredimethylester (1,136 g; 8,6 mmol) und Kaliumcarbonat (5,35 g; 38,7 mmol) wird das Reaktionsgemisch über Nacht unter Rückfluß gerührt. Nach dem Abkühlen werden die nicht gelösten Salze abgesaugt und das Filtrat i.V. eingeengt. Der Rückstand wird an Kieselgel mit Toluol : Ethylacetat (20: 1) chromatographiert.
Ausbeute: 1,95 g (35% d.Th.)
R_{f} = 0,45 (Toluol : Ethylacetat = 20:1)
MS (DCI/NH₃): m/z = 534 (M+NH₄)

Als Nebenprodukt wird 4,4,4-Trifluor-1-butansulfonsäure 3-(1-Brom-2,2-bismethoxycarbonyl-indanyl-4-oxy)-phenylester (0,.82 g; 16% d.Th.; R_{f} = 0,52 (Toluol : Ethylacetat = 20:1); MS (DCI / NH₃): m/z = 612, 614 (M+NH₄) erhalten.

### Beispiel 8A

### (R,S)-4,4,4-Trifluor-1-butansulfonsäure-3-(2-hydroxycarbonyl-indanyl-4-oxy)-phenylester

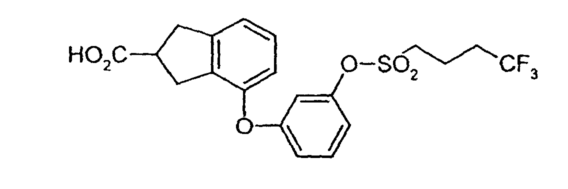

Eine Lösung von Beispiel 7A (66,0 g; 128 mmol) in Essigsäure (900 ml) und Bromwasserstoff; 48%ig in Wasser (350 ml) wird 5,5 h unter Argon zum Rückfluß erhitzt. Anschließend wird der Ansatz im Vakuum eingeengt und der Rückstand in Ethylacetat aufgenommen und mit Wasser (1 x 250 ml; 2 x 150 ml) gewaschen. Die organische Phase wird getrockent (Na₂SO₄) und im Vakuum eingeengt.
Ausbeute: 55,4 g (88% d.Th.)
Gehalt nach HPLC : 90 Fl-%
MS-(DCI, NH₃): m/z = 462 (M+NH₄)

### Beispiel 9A

### (R,S)-4,4,4-Trifluor-1-butansulfonsäure-3-(2-hydroxymethyl-indanyl-4-oxy)-phenylester

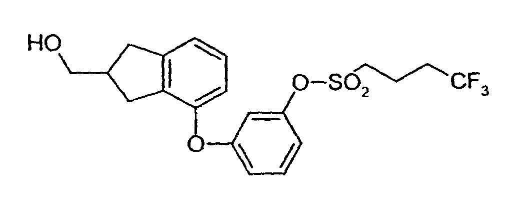

Zur Lösung von Beispiel 8A (53,9 g; 109 mmol; 90%ig nach HPLC) in THF (1500 ml) tropft man Boran-Dimethylsulfid-Komplex, 2 M in THF (63,0 ml; 126 mmol) bei RT unter Argon zu und läßt noch 1 h bei RT nachrühren. Nach Zugabe von Wasser (8 ml) wird das THF i.V. abgezogen, der Rückstand in Ethylacetat (800 ml) aufgenommen und mit Wasser (2 x 150 ml) gewaschen. Die organische Phase wird getrocket (MgSO₄) und im Vakuum eingeengt. Der Rückstand wird an Kieselgel mit Toluol : Ethylacetat (10:1) chromatographiert.
Ausbeute: 34,0 g (72% d.Th.)
R_{f} = 0,39 (Tol : EE = 3:1)
MS (DCI, NH₃): m/z = 448 (M+NH₄)

### Beispiel 10A und 11 A

### (S)- und (R)-1-(4,4,4-Trifluor-1-butansulfonsäure-3-(2-hydroxymethyl-indanyl-4-oxy)-phenylester

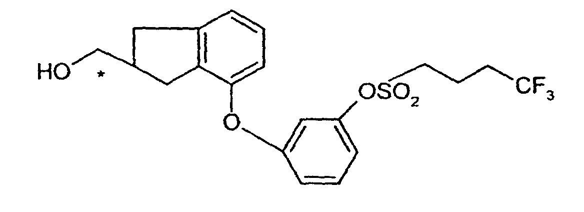

### (S)-(+)-Enantiomer A (Beispiel 10A) und (R)-(-)-Enantiomer B (Beispiel 11A)

Die Verbindung aus Beispiel 9A (490 mg; 1,14 mmol) wird mittels präparativer HPLC (Chiracel OD, 10 µm, 250 x 20 mm, Fluß 10 ml/min, Laufmittel 80 %, Petroleumbenzin 40-70°C / 20 % Isopropanol, T = 10°C) in das (S)-Enantiomere (Beispiel 10A) und das (R)-Enantiomere (Beispiel 11A) getrennt.

### Beispiel 10A:

Ausbeute: 111 mg (23 % d. Th.)
Smp.: 60-61°C
Retentionszeit: 12,5 min
[α]_{D}²⁰ (c = 1, MeOH) = + 10,70

Die absolute (S)-Konfiguration von Beispiel 10A wurde durch Röntgenstrukturanalyse ermittelt.

### Beispiel 11A:

Ausbeute: 105 mg (21 % d. Th.)
Smp.: 60-61°C
Retentionszeit: 15,4 min
[α]_{D}²⁰ (c = 1, MeOH) = - 10,35

### Herstellungsbeispiele

### Beispiel 1

### (R)-4,4,4-Trifluor-1-butansulfonsäure-3-[2-(N-tert.-butyloxycarbonylglycinyl)-oxymethyl-indanyl-4-oxy]-phenylester

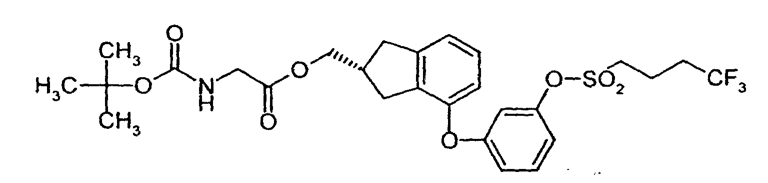

Zur Lösung des Beispiels 11A (565 mg; 1,31 mmol) in Dichlormethan (20 ml) gibt man unter Eiskühlung unter Argon N-tert.Butyloxycarbonylglycin (230 mg; 1,31 mmol) , N-Ethyl-N'-3-(dimethylaminopropyl)-carbodiimid Hydrochlorid (277 mg; 1,44 mmol) und 9-Dimethylaminopyridin (16 mg; 0,13 mmol) und läßt 18 h bei RT nachrühren. Anschließend wird der Ansatz mit Dichlormethan (30 ml) vedünnt, mit Wasser (60 ml), ges. wäßriger NaHCO₃-Lösung (60 ml) und Wasser (60 ml) gewaschen, getrocknet (Na₂SO₄) und i.V. eingeengt. Der Rückstand wird an Kieselgel mit Tol : EE = 10:1 chromatographiert.
Ausbeute: 651 mg (85% d.Th.)
R_{f} = 0,39 (Tol : EE = 5:1)
MS (DCI, NH₃): m/z = 605 (M+NH₄)

### Beispiel 2

### (R)-4,4,4-Trifluor-1-butansulfonsäure-3-[2-(7-N-tert.-butyloxycarbonylaminoheptanoyloxymethyl)-indanyl-4-oxy]-phenylester

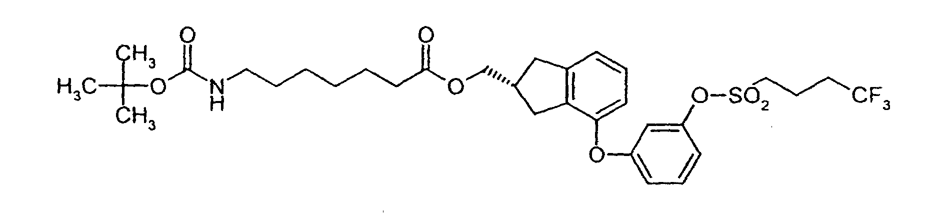

Die Herstellung erfolgt in Analogie zur Herstellung des Beispiels 12A ausgehend von Beispiel 11A (196 mg; 0,46 mmol) und 7-N-tert.-Butyloxycarbonylaminoheptansäure (285 mg; 1,16 mmol).
Ausbeute: 265 mg (87% d.Th.)
R_{f} = 0,19 (Tol : EE = 10:1)
MS (DCI, NH₃): m/z = 675 (M+NH₄)

### Beispiel 3

### (R)-4,4,4-Trifluor-1-butansulfonsäure-3-[2-(3-N-tert.-butyloxycarbonylaminopropanoyloxymethyl)-indanyl-4-oxy]-phenylester

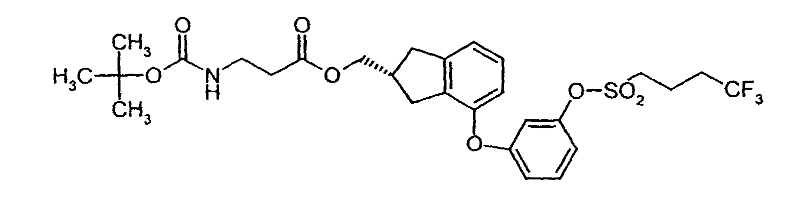

Die Herstellung erfolgte in Analogie zur Herstellung des Beispiels 1 ausgehend von Beispiel 11A (600 mg; 1,39 mmol) und N-tert.Butyloxycarbonyl-β-alanin (290 mg; 1,53 mmol).
Ausbeute: 499 mg (59% d.Th.)
R_{f} = 0,41 (Tol : EE = 5:1)
MS (ESI): m/z 602 (M+H)

### Beispiel 4

### (R)-4,4,4-Trifluor-1-butansulfonsäure-3-[2-((S)-N-tert.-butyloxycarbonylvalinyl)-indanyl-4-oxy]-phenylester

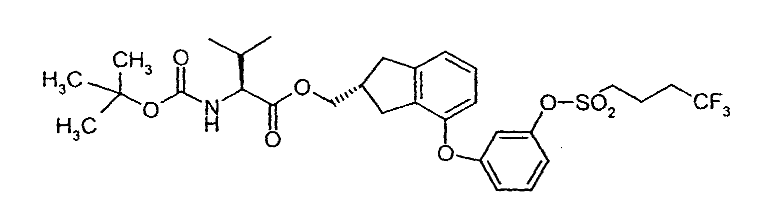

Die Herstellung erfolgt in Analogie zur Herstellung des Beispiels 1 ausgehend von Beispiel 11 A (600 mg; 1,39 mmol) und N-tert.Butyloxycarbonyl-(S)-valin (394 mg; 1,86 mmol).
Ausbeute: 745 mg (85% d.Th.)
R_{f} = 0,58 (Tol : EE = 5:1)
MS (ESI): m/z 652 (M+Na)

### Beispiel 5

### (R)-4,4,4-Trifluor-1-butansulfonsäure-3-(2-glycinyl-oxymethyl-indanyl-4-oxy)-phenylester Hydrochlorid

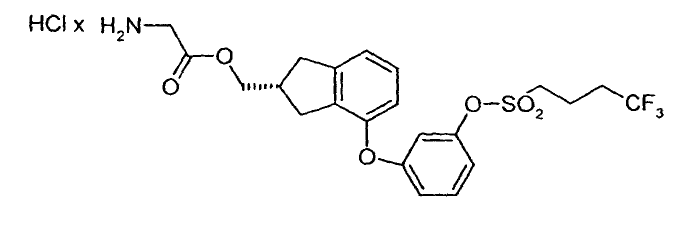

Zu einer Lösung von Beispiel 1 (537 mg; 0.91 mmol) in 1,4-Dioxan (4 ml) wird eine Lösung von 4 N HCl in 1,4-Dioxan (5 ml) bei RT unter Argon getropft. Man läßt über Nacht bei RT rühren, zieht das Lösungsmittel i.V. ab und verreibt den Rückstand mit Diethylether/Petrolether.
Ausbeute: 479 mg (100% d. Th.)
MS (ESI): m/z = 488 (M+H)
R_{f} = 0.21 (Dichlormethan : Methanol : Triethylamin = 20 : 1 : 0,2)

### Beispiel 6

### (R)-4,4,4-Trifluor-1-butansulfonsäure-3-[2-(7-aminoheptanoyloxymethyl)-indanyl-4-oxy]-phenylester Hydrochlorid

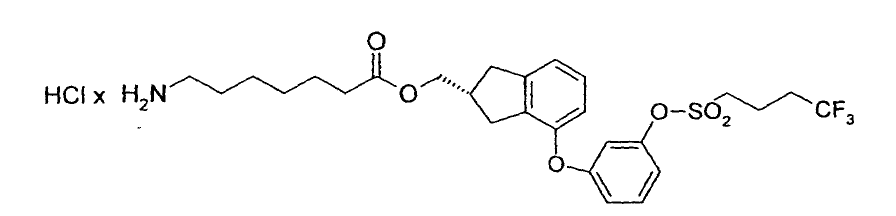

Die Herstellung erfolgt in Analogie zur Herstellung des Beispiels 5 ausgehend von Beispiel 2 (225 mg; 0.34 mmol).
Ausbeute: 202 mg (99% d. Th.)
MS (ESI): m/e = 558 (M+H)
R_{f} = 0.19 (Dichlormethan : Methanol : Triethylamin = 20 : 1 : 0,2)

### Beispiel 7

### (R)-4,4,4-Trifluor-1-butansulfonsäure-3-[2-(3-aminopropanoyloxymethyl)-indanyl-4-oxy]-phenylester Hydrochlorid

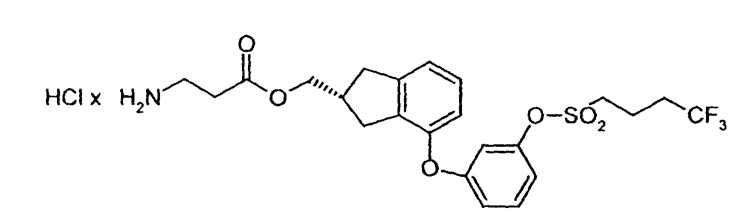

Die Herstellung erfolgt in Analogie zur Herstellung des Beispiels 5 augehend von Beispiel 3 (417mg; 0,69mmol).
Ausbeute: 374mg (100% d. Th.)
MS (DCI/NH₃): m/z = 502 (M+H)
R_{f} = 0.19 (Dichlormethan : Methanol : Triethylamin = 20 : 1 : 0,2)

### Beispiel 8

### (R)-4,4,4-Trifluor-1-butansulfonsäure-3-[2-((S)-valinyloxymethyl)-indanyl-4-oxy]-phenylester Hydrochlorid

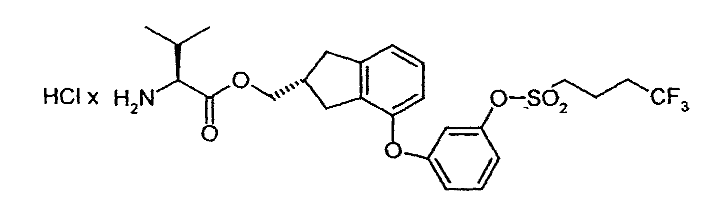

Die Herstellung erfolgte in Analogie zur Herstellung des Beispiels 5 ausgehend von Beispiel 4 (706mg; 1, 12mmol).
Ausbeute: 621 mg (98% d. Th.)
MS (DCI/NH₃): m/z = 530 (M+H)
R_{f} = 0.30 (Dichlormethan : Methanol : Triethylamin = 20 : 1 : 0,2)

## Patentansprüche

1. Verbindungen der allgemeinen Formel (I)
R¹―A―D―E―G―L―R² (I)
in welcher
R¹ für einen Rest der Formel 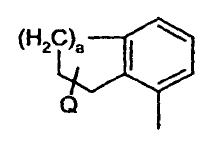 steht,
worin
a eine Zahl 1 oder 2 bedeutet,
und wobei der oben aufgeführte Phenylrest gegebenenfalls mit einem oder mehreren, gleichen oder verschiedenen Substituenten substituiert ist, die aus der Gruppe ausgewählt sind, die besteht aus:
Chlor, Fluor, Hydroxyl, (C₁-C₃)-Alkoxy oder (C₁-C₄)-Alkyl, das seinerseits durch Hydroxy substituiert sein kann,
Q einen Rest der Formel 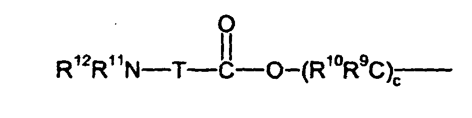 bedeutet,
worin
c eine Zahl 1, 2, 3 oder 4 bedeutet,
R⁹ und R¹⁰ gleich oder verschieden sind und Wasserstoff oder (C₁-C₃)-Alkyl bedeuten,
T einen Rest der Formel -(CH₂)_{d}- bedeutet,
worin
d eine Zahl 1, 2, 3, 4, 5 oder 6 bedeutet,
oder
T für einen Teil eines Aminosäurerestes der Formel 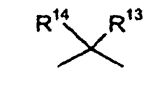 steht,
worin
R¹³ Wasserstoff oder Methyl bedeutet
und
R¹⁴ Cyclopentyl, Cyclohexyl, Phenyl oder Wasserstoff bedeutet, oder (C₁-C₄)-Alkyl bedeutet,
wobei das (C₁-C₄)-Alkyl gegebenenfalls durch Methlythio, Hydroxy, Mercapto, Guanidyl oder durch eine Gruppe der Formel -NR¹⁵R¹⁶ substituiert ist,
worin
R¹⁵ und R¹⁶ unabhängig voneinander Wasserstoff, (C₁-C₈)-Alkyl oder Phenyl bedeuten
oder das (C₁-C₄)-Alkyl gegebenenfalls durch Cyclopentyl, Cyclohexyl oder Phenyl substituiert ist, das seinerseits durch Hydroxy, Fluor, Chlor oder (C₁-C₃)-Alkoxy oder Amino substituiert ist,
und
R¹¹ und R¹² gleich oder verschieden sind und Wasserstoff oder (C₁-C₆)-Alkyl bedeuten,
oder
R¹¹ und R¹² gemeinsam mit dem Stickstoffatom einen Morpholinylring bilden,
A und E für eine Bindung stehen,
D für ein Sauerstoffatom steht,
G für zweifach gebundenes Phenyl steht, das gegebenenfalls mit einem oder mehreren, gleichen oder verschiedenen Substituenten substituiert ist, die aus der Gruppe ausgewählt sind, die besteht aus:
Hydroxy, Trifluormethyl, Carboxyl, Fluor, Chlor, Brom, (C₁-C₃)-Alkyl, Hydroxy(C₁-C₃)alkyl oder (C₁-C₃)-Alkoxy,
L für einen Rest der Formel 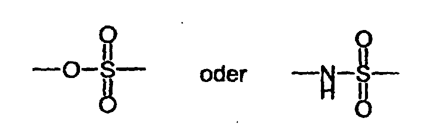 steht,
wobei die Anbindung der Reste an G linksbündig erfolgt,
R² für (C₁-C₈)-Alkyl steht, das gegebenenfalls mit einem oder mehreren, gleichen oder verschiedenen Substitutenen substituiert ist, die aus der Gruppe ausgewählt sind, die besteht aus:
Fluor, Chlor, Brom, Phenyl, Trifluormethyl oder Trifluormethylsubstituiertem (C₁-C₄)-Alkoxy,
und deren pharmazeutisch verträgliche Salze.

2. Verbindungen der allgemeinen Formel (I), gemäß den Anspruch 1,
in welcher
R¹ für einen Rest der Formel 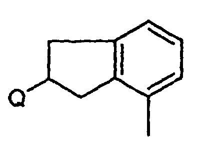 steht,
worin
Q einen Rest der Formel 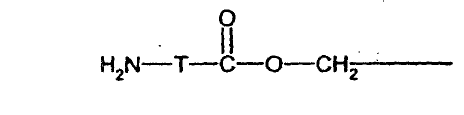 bedeutet,
worin
T einen Rest der Formel -(CH₂)_{d}- bedeutet,
worin
d eine Zahl 1, 2, 3, 4, 5 oder 6 bedeutet,
oder
T einen Teil eines Aminosäurerestes der Formel 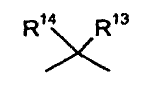 bedeutet,
worin
R¹³ Wasserstoff bedeutet,
und
R¹⁴ Wasserstoff, (C₁-C₄)-Alkyl, Benzyl oder einen Rest der Formel -CH₂OH bedeutet,
A und E für eine Bindung stehen,
D für ein Sauerstoffatom steht,
G für Phenyl steht, das gegebenenfalls durch Fluor, Chlor oder Brom substituiert ist,
L für einen Rest der Formel 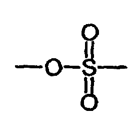 steht,
wobei die Anbindung des Restes an G linksbündig erfolgt,
R² für (C₁-C₄)-Alkyl steht, das gegebenenfalls durch Fluor oder . Trifluormethyl substituiert ist,
und deren pharmazeutisch verträgliche Salze.

3. Verbindungen ausgewählt aus der Gruppe
(R)-4,4,4-Trifluor-1-butansulfonsäure-3-(2-glycinyl-oxymethyl-indanyl-4-oxy)-phenylester 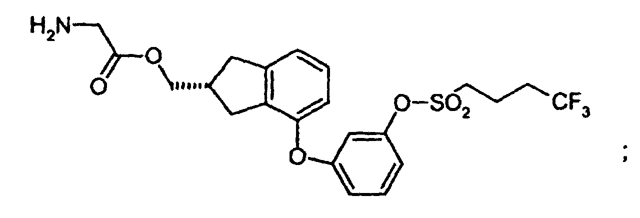 (R)-4,4,4-Trifluor-1-butansulfonsäure-3-[2-(7-aminoheptanoyloxymethyl)-indanyl-4-oxy]-phenylester 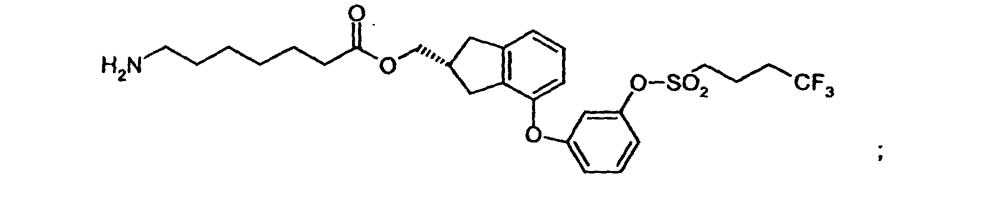 (R)-4,4,4-Trifluor-1-butansulfonsäure-3-[2-(3-aminopropanoyloxymethyl)-indanyl-4-oxy]-phenylester 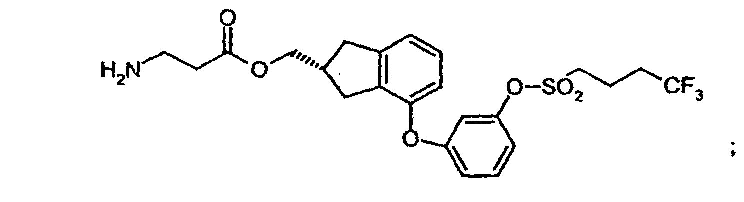 und
(R)-4,4,4-Trifluor-1-butansulfonsäure-3-[2-((S)-valinyloxymethyl)-indanyl-4-oxy]-phenylester 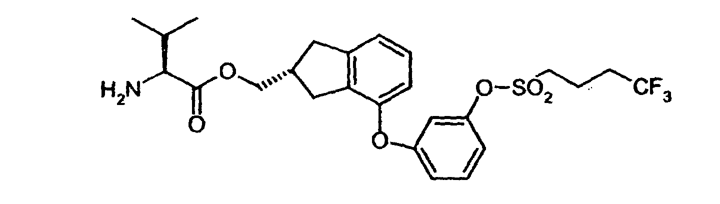 und deren pharmazeutisch verträgliche Salze.

4. Verfahren zur Herstellung der erfindungsgemäßen Verbindungen der allgemeinen Formel (I), **dadurch gekennzeichnet, daß** man Verbindungen der allgemeinen Formel (II)
R^{1'}-A-D-E-G-L-R² (II)
worin
A, D,E, G, L, R², die oben angegebene Bedeutung haben
und
R^{1'} für einen Rest der Formel 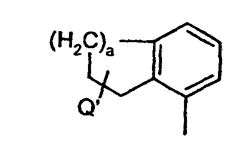 steht,
worin
a eine Zahl 1 oder 2 bedeutet,
und wobei der oben aufgeführten Phenylrest gegebenenfalls mit einem oder mehreren, gleichen oder verschiedenen Substituenten substituiert ist, die aus der Gruppe ausgewählt sind, die besteht aus:
Chlor, Fluor Hydroxy, Phenyl, (C₁-C₃)-Alkoxy, (C₁-C₄)-Alkyl, das seinerseits durch Hydroxy substituiert sein kann,
Q' einen Rest der Formel HO-(R¹⁰R⁹C)_{c})- bedeutet,
worin
c, R⁹ und R¹⁰ die oben angegebene Bedeutung haben,
mit Verbindungen der allgemeinen Formel (III) 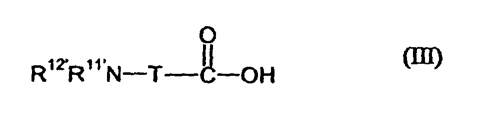 in welcher
R^{11'} für Wasserstoff steht
und
R^{12'} für eine der oben aufgeführten Aminoschutzgruppen, vorzugsweise für tert.Butyloxycarbonyl steht,
in inerten Lösemitteln, gegebenenfalls in Anwesenheit einer Base und eines Hilfsstoffes,
umsetzt,
und die Aminoschutzgruppe nach üblichen Methoden abspaltet,
und dann gegebenenfalls die Aminogruppe mit einem Aldehyd oder Keton reduktiv alkyliert oder dialkyliert,
oder mit einem Halogenid alkyliert oder dialkyliert,
und gegebenenfalls in Abhängigkeit der oben aufgeführten Substituenten nach üblichen Methoden wie beispielsweise einer Alkylierung oder Veresterung Derivatisierungen anschließt.

5. Verfahren zur Herstellung von Verbindungen der allgemeinen Formel (IIk) 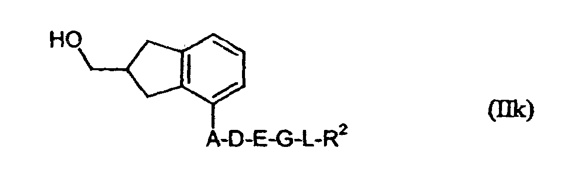 in welcher
A, D, E, G, L und R² die oben angegebene Bedeutung haben,
**dadurch gekennzeichnet, daß** man die Verbindungen der allgemeinen Formel (IIz) 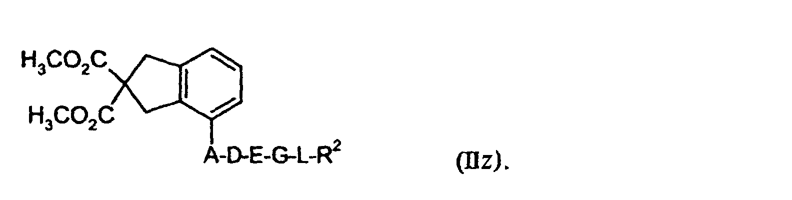 in welcher
A, D, E, G, L und R2 die oben angegebene Bedeutung haben,
durch Einsatz von HBr und Essigsäure in die Verbindungen der allgemeinen Formel (IIm) 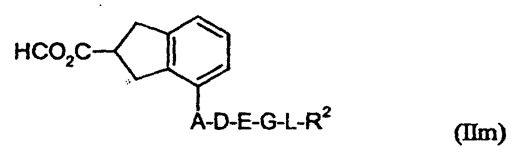 in welcher
A, D, E, G, L und R2 die oben angegebene Bedeutung haben,
überführt,
und in einem letzten Schritt eine Reduktion mit BH₃ x S(CH₃)₂ in Tetrahydrofuran durchführt,
und im Fall der reinen Enantiomeren eine HPLC-Trennung nach üblichen Methoden durchführt.

6. Pharmazeutische Zubereitungen, die als aktiven Bestandteil mindestens eine Verbindung gemäß irgendeinem der Ansprüche 1 bis 3 in Zusammenmischung mit mindestens einem pharmazeutisch verträglichen nichtgiftigen Träger oder Exzipienten umfaßt.

7. Verbindungen nach irgendeinem der Ansprüche 1 bis 3 zur Verwendung als Medikament in der Behandlung von Menschen und Tieren.

8. Verwendung der Verbindungen gemäß irgendeinem der Ansprüche 1 bis 3 für die Herstellung eines Medikamentes zur Prävention und/oder Behandlung neurodegenerativer Erkrankungen.

9. Verwendung der Verbindungen gemäß irgendeinem der Ansprüche 1 bis 3 für die Herstellung eines Medikamentes zur Prävention und/oder Behandlung von cerebralen Ischämien und Schädel/Hirn-Trauma.

10. Verwendung der Verbindungen gemäß irgendeinem der Ansprüche 1 bis 3 für die Herstellung eines Medikamentes zur Behandlung von Schmerzzuständen, Emesis, Übelkeit, Glaukom, Asthma, Anorexie, Konvulsionen, Rheuma, Sedation und Bewegungsstörungen.

11. Verwerdung der Verbindungen gemäß irgendeinem der Ansprüche 1 bis 3 für die Herstellung eines Medikamentes zur Behandlung von bakteriellen oder viralen Infektionen, Autoimmunerkrankungen, entzündlicher oder autoimmunologisch bedingter Erkrankungen der Gelenke des Knochen- und Muskelapparates, der inneren und äußeren Organe, des zenralen Nervensystems, der Sinnesorgane und des blutbildenden Systems bei Mensch und Tier.

12. Verwendung der Verbindungen gemäß irgendeinem der Ansprüche 1 bis 3 für die Herstellung eines Medikamentes zur Behandlung von Migräne und Spastizität.

## Claims

1. Compounds of the general formula (I)
R¹-A-D-E-G-L-R² (I)
in which
R¹ represents a radical of the formula 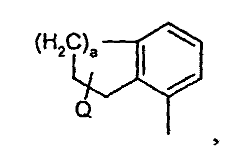
in which
a represents a number 1 or 2,
and where the phenyl radical listed above is optionally substituted by one or more identical or different substituents selected from the group consisting of:
chlorine, fluorine, hydroxyl, (C₁-C₃)-alkoxy or (C₁-C₄)-alkyl, which for its part may be substituted by hydroxyl,
Q represents a radical of the formula 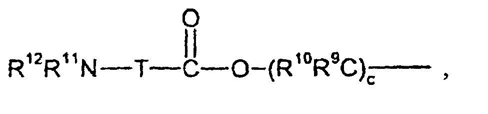
in which
c represents a number 1, 2, 3 or 4,
R⁹ and R¹⁰ are identical or different and represent hydrogen or (C₁-C₃)-alkyl,
T represents a radical of the formula -(CH₂)_{d}-,
in which
d represents a number 1, 2, 3, 4, 5 or 6,
or
T represents a moiety of an amino acid radical of the formula 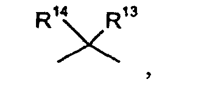
in which
R¹³ represents hydrogen or methyl
and
R¹⁴ represents cyclopentyl, cyclohexyl, phenyl or hydrogen, or represents (C₁-C₄)-alkyl,
where the (C₁-C₄)-alkyl is optionally substituted by methlythio, hydroxyl, mercapto, guanidyl or by a group of the formula -NR¹⁵R¹⁶,
in which
R¹⁵ and R¹⁶ independently of one another represent hydrogen, (C₁-C₈)-alkyl or phenyl
or the (C₁-C₄)-alkyl is optionally substituted by cyclopentyl, cyclohexyl or phenyl, which for its part is substituted by hydroxyl, fluorine, chlorine or (C₁-C₃)-alkoxy or amino,
and
R¹¹ and R¹² are identical or different and represent hydrogen or (C₁-C₆)-alkyl,
or
R¹¹ and R¹² together with the nitrogen atom form a morpholinyl ring,
A and E represent a bond,
D represents an oxygen atom,
G represents doubly attached phenyl which is optionally substituted by one or more identical or different substituents selected from the group consisting of:
hydroxyl, trifluoromethyl, carboxyl, fluorine, chlorine, bromine, (C₁-C₃)-alkyl, hydroxy(C₁-C₃)alkyl or (C₁-C₃)-alkoxy,
L represents a radical of the formula 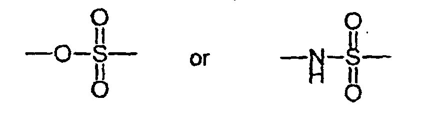 where the left-hand side of the radicals is attached to G,
R² represents (C₁-C₈)-alkyl, which is optionally substituted by one or more identical or different substituents selected from the group consisting of:
fluorine, chlorine, bromine, phenyl, trifluoromethyl or trifluoromethyl-substituted (C₁-C₄)-alkoxy,
and pharmaceutically acceptable salts thereof.

2. Compounds of the general formula (I), according to Claim 1,
in which
R¹ represents a radical of the formula 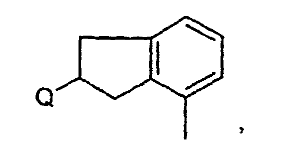
in which
Q represents a radical of the formula 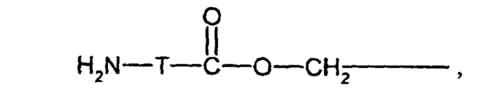
in which
T represents a radical of the formula -(CH₂)_{d}-,
in which
d represents a number 1, 2, 3, 4, 5 or 6,
or
T represents a moiety of an amino acid radical of the formula 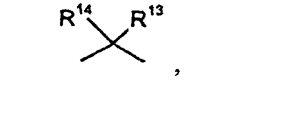 in which
R¹³ represents hydrogen,
and
R¹⁴ represents hydrogen, (C₁-C₄)-alkyl, benzyl or a radical of the formula -CH₂OH,
A and E represent a bond,
D represents an oxygen atom,
G represents phenyl, which is optionally substituted by fluorine, chlorine or bromine,
L represents a radical of the formula 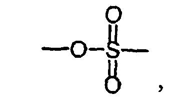 where the left-hand side of the radical is attached to G,
R² represents (C₁-C₄)-alkyl, which is optionally substituted by fluorine or trifluoromethyl,
and pharmaceutically acceptable salts thereof.

3. Compounds selected from the group consisting of
(R)-3-(2-glycinyl-oxymethyl-indanyl-4-oxy)-phenyl 4,4,4-trifluoro-1-butanesulphonate 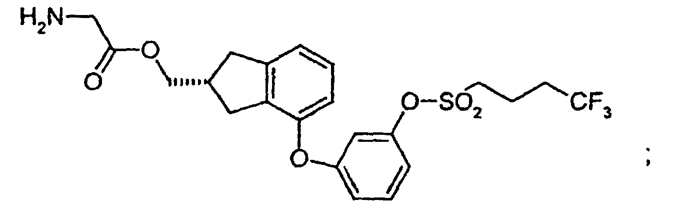 (R)-3-[2-(7-aminoheptanoyloxymethyl)-indanyl-4-oxy]-phenyl 4,4,4-trifluoro-1-butanesulphonate 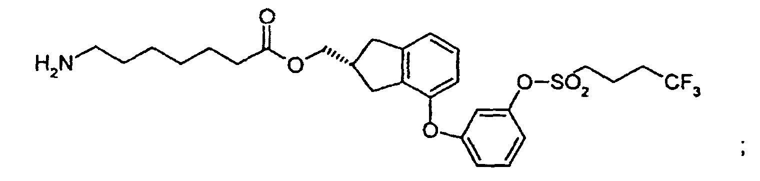 (R)-3-[2-(3-aminopropanoyloxymethyl)-indanyl-4-oxy)-phenyl 4,4,4-trifluoro-1-butanesulphonate 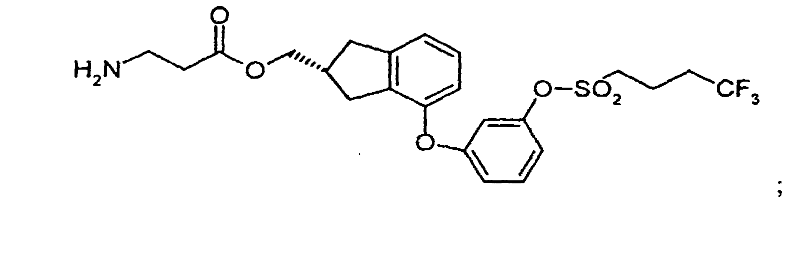 and
(R)-3-[2-((S)-valinyloxymethyl)-indanyl-4-oxy]-phenyl 4,4,4-trifluoro-1-butanesulphonate 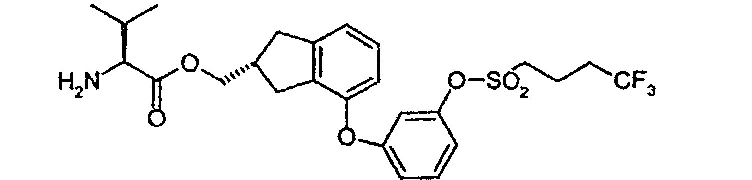 and pharmaceutically acceptable salts thereof.

4. Process for preparing the compounds of the general formula (I) according to the invention, **characterized in that** compounds of the general formula (II)
R^{1'}-A-D-E-G-L-R² (II)
in which
A, D, E, G, L, R² have the abovementioned meaning
and
R^{1'} represents a radical of the formula 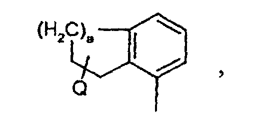
in which
a represents a number 1 or 2,
and where the phenyl radicals listed above is optionally substituted by one or more identical or different substituents selected from the group consisting of:
chlorine, fluorine, hydroxyl, phenyl, (C₁-C₃)-alkoxy, (C₁-C₄)-alkyl, which for its part may be substituted by hydroxyl,
Q' represents a radical of the formula HO-(R¹⁰R⁹C)_{c})-,
in which
c, R⁹ and R¹⁰ have the meaning given above,
are reacted with compounds of the general formula (III) 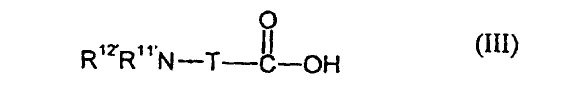 in which
R^{11'} represents hydrogen
and
R^{12'} represents one of the amino protective groups listed above, preferably represents tert-butyloxycarbonyl,
in inert solvents, if appropriate in the presence of a base and an auxiliary, and the amino protective group is removed by customary methods,
and the amino group is then, if appropriate, reductively alkylated or dialkylated with an aldehyde or ketone,
or alkylated or dialkylated with a halide,
followed, if appropriate, by derivatizations according to customary methods, such as, for example, an alkylation or esterification, depending on the substituents listed above.

5. Process for preparing compounds of the general formula (IIk) 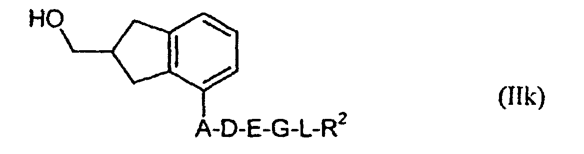 in which
A, D, E, G, L and R² have the meaning given above,
**characterized in that** the compounds of the general formula (IIz) 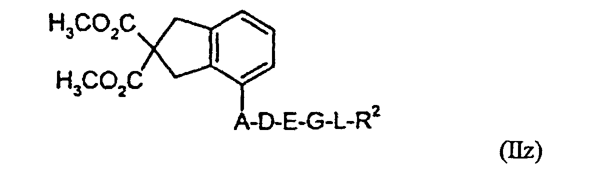 in which
A, D, E, G, L and R2 have the meaning given above
are converted, by using HBr and acetic acid, into the compounds of the general formula (IIm) 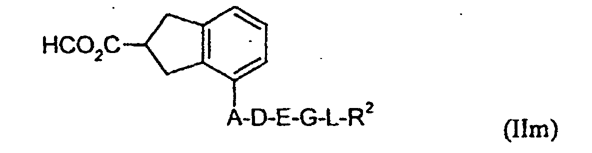 in which
A, D, E, G, L and R2 have the meaning given above,
and, in a last step, a reduction with BH₃ x S(CH₃)₂ in tetrahydrofuran is carried out,
and, in the case of the pure enantiomers, an HPLC separation by customary methods is carried out.

6. Pharmaceutical preparations which comprise, as active component, at least one compound according to any of Claims 1 to 3 in combination with at least one pharmaceutically acceptable non-toxic vehicle or excipient.

7. Compounds according to any of Claims 1 to 3 for use as medicaments in the treatment of humans and animals.

8. Use of the compounds according to any of Claims 1 to 3 for preparing a medicament for the prevention and/or treatment of neurodegenerative disorders.

9. Use of the compounds according to any of Claims 1 to 3 for preparing a medicament for the prevention and/or treatment of cerebral ischaemias and craniocerebral trauma.

10. Use of the compounds according to any of Claims 1 to 3 for preparing a medicament for the treatment of states of pain, emesis, nausea, glaucoma, asthma, anorexia, convulsions, rheumatism, sedation and mobility disorders.

11. Use of the compounds according to any of Claims 1 to 3 for preparing a medicament for the treatment of bacterial or viral infections, autoimmune diseases, inflammatory or autoimmunologically related diseases of the joints of the bone and muscle apparatus, of the internal and external organs, of the central nervous system, of the sense organs and of the haematogenic system in humans and animals.

12. Use of the compounds according to any of Claims 1 to 3 for preparing a medicament for the treatment of migraine and spasticity.

## Revendications

1. ComposéS de formule générale (I)
R¹-A-D-E-G-L-R² (I)
dans laquelle
R¹ représente un reste de formule 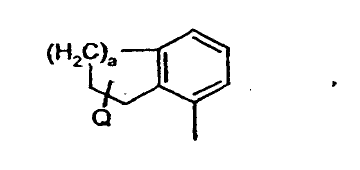
dans laquelle
a représente le nombre 1 ou 2,
et où le reste phényle représenté ci-dessus est éventuellement substitué avec un ou plusieurs substituants identiques ou différents, qui sont choisis dans le groupe constitué par les radicaux :
chloro, fluoro, hydroxyle, alkoxy en C₁ à C₃ ou alkyle en C₁ à C₄, qui peut lui-même être substitué par un radical hydroxy,
Q est un reste de formule 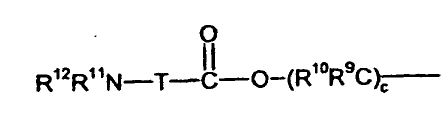
dans laquelle
c représente le nombre 1, 2, 3 ou 4,
R⁹ et R¹⁰ sont identiques ou différents et représentent l'hydrogène ou un reste alkyle en C₁ à C₃,
T est un reste de formule -(CH₂)_{d}-,
dans lequel
d représente le nombre 1, 2, 3, 4, 5 ou 6,
ou bien
T est une partie d'un reste d'aminoacide, de formule 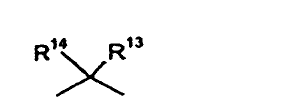
dans laquelle
R¹³ représente l'hydrogène ou un radical méthyle
et
R¹⁴ est un radical cyclopentyle, cyclohexyle, phényle ou l'hydrogène, ou représente
un radical alkyle en C₁ à C₄,
ce radical alkyle en C₁ à C₄ étant éventuellement substitué par un radical méthylthio, hydroxy, mercapto, guanidyle ou par un groupe de formule -NR¹⁵R¹⁶
dans laquelle
R¹⁵ et R¹⁶ représentent indépendamment l'un de l'autre l'hydrogène, un radical alkyle en C₁ à C₈ ou phényle,
ou bien le radical alkyle en C₁ à C₄ est éventuellement substitué par un radical cyclopentyle, cyclohexyle ou phényle qui est lui-même substitué par un radical hydroxy, fluoro, chloro ou alkoxy en C₁ à C₃ ou un radical amino,
et
R¹¹ et R¹² sont identiques ou différents et représentent l'hydrogène ou un radical alkyle en C₁ à C₆,
ou bien
R¹¹ et R¹² forment conjointement avec l'atome d'azote un noyau morpholinyle,
A et E représentent une liaison,
D est un atome d'oxygène,
G représente un reste phényle doublement lié, qui porte éventuellement un ou plusieurs substituants identiques ou différentes choisis dans le groupe constitué par des radicaux :
hydroxy, trifluorométhyle, carboxyle, fluoro, chloro, bromo, alkyle en C₁ à C₃, hydroxyalkyle en C₁ à C₃ ou alkoxy en C₁ à C₃,
L est un reste de formule 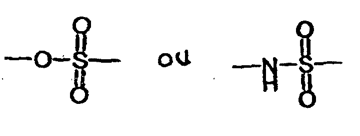
la liaison des restes à G s'effectuant du côté gauche,
R² est un reste alkyle en C₁ à C₈ qui porte éventuellement un ou plusieurs substituants identiques ou différents choisis dans le groupe constitué par les radicaux :
fluoro, chloro, bromo, phényle, trifluorométhyle ou alkoxy en C₁ à C₄ à substituant trifluorométhyle,
et leurs sels acceptables du point de vue pharmaceutique.

2. Composés de formule générale (I) suivant la revendication 1,
formule dans laquelle
R¹ représente un reste de formule 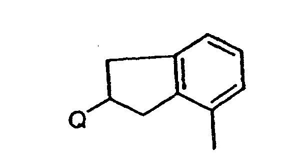
où
Q est un reste de formule 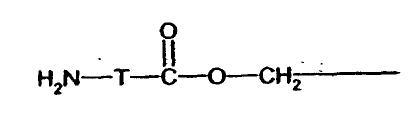
dans laquelle
T est un reste de formule -(CH₂)_{d}-,
d désignant le nombre 1,2,3,4, 5 ou 6,
ou bien
T est une partie d'un reste d'aminoacide de formule 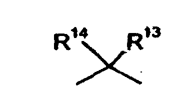
dans laquelle
R¹³ représente l'hydrogène,
et
R¹⁴ est l'hydrogène, un reste alkyle en C₁ à C₄, benzyle
ou un reste de formule -CH₂OH,
A et E représentent une liaison,
D est un atome d'oxygène,
G est un reste phényle qui est éventuellement substitué par du fluor, du chlore ou du baume,
L est un reste de formule 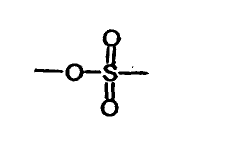 la liaison du reste à G étant effectuée du côté gauche,
R² est un reste alkyle en C₁ à C₄ qui est éventuellement substitué par du fluor ou par un radical trifluorométhyle,
et leurs sels acceptables du point de vue pharmaceutique.

3. Composés choisis dans le groupe :
ester (R)3-(2-glycinyloxyméthylindanyl-4-oxyphénylique d'acide 4,4,4-trifluoro-1-butanesulfonique 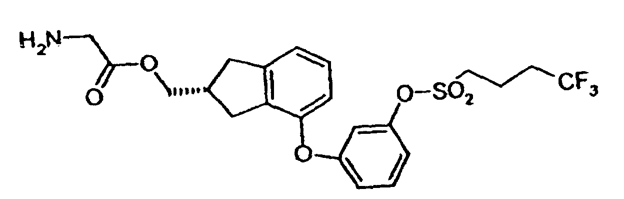
ester (R)3-[2(7-aminoheptanoyloxyméthyl)-indanyl-4-oxyphénylique d'acide 4,4,4-trifluoro-1-butanesulfonique 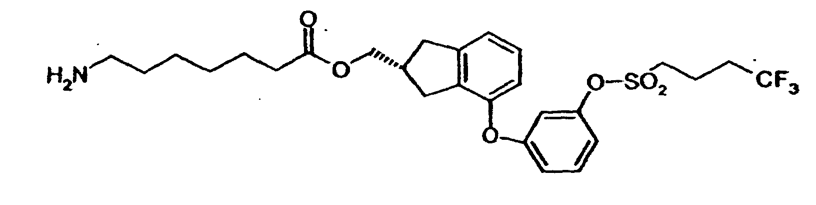
ester (R)3-[2(3-aminopropanoyloxyméthyl)-indanyl-4-oxyphénylique d'acide 4,4,4-trifluoro-1-butanesulfonique 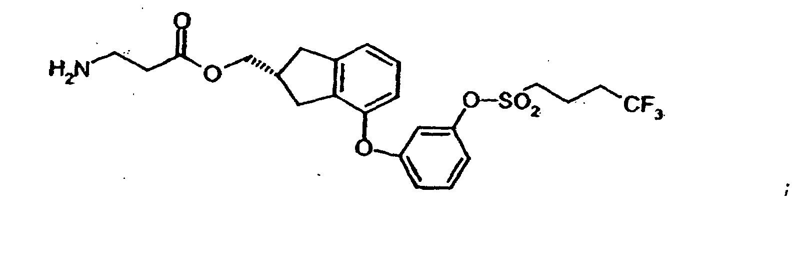 et
ester (R)3-[2((S)-valinyloxyméthyl)-indanyl-4-oxyphénylique d'acide 4,4,4-trifluoro-1-butanesulfonique 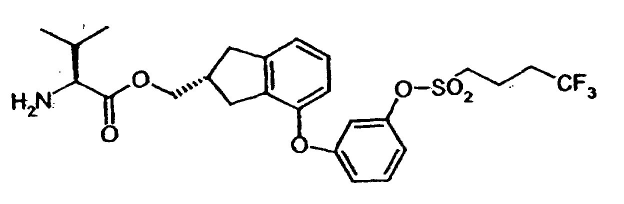
et leurs sels acceptables du point de vue pharmaceutique.

4. Procédé de production des composés conformes à l'invention de formule générale (I), **caractérisé en ce qu'**on fait réagir des composés de formule générale (II)
R^{1'}-A-D-E-G-L-R² (II)
dans laquelle
A, D, E, G, L, R² ont la définition indiquée ci-dessus
et
R^{1'} représente un reste de formule
où 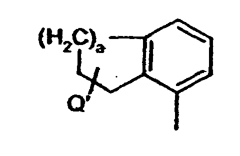
a représente le nombre 1 ou 2,
et le reste phényle représenté ci-dessus porte éventuellement un ou plusieurs substituants, identiques ou différents, choisis dans le groupe constitué par les radicaux :
chloro, fluoro, hydroxy, phényle, alkoxy en C₁ à C₃, alkyle en C₁ à C₄ qui peut lui-même être substitué par un radical hydroxy,
Q' est un reste de formule HO-(R¹⁰R⁹C)_{c})-
dans lequel
c, R⁹ et R dont la définition indiquée ci-dessus,
avec des composés de formule générale (III) 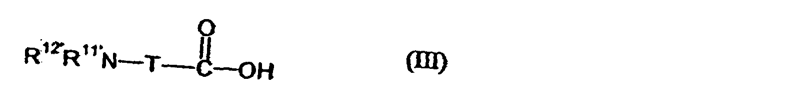 dans laquelle
R^{11'} représente l'hydrogène
et
R^{12'} représente l'un des groupes protégeant la fonction amino énumérés ci-dessus, avantageusement le groupe tertiobutyloxycarbonyle,
dans des solvants inertes, éventuellement en présence d'une base et d'une substance auxiliaire,
et on élimine le groupe protégeant la fonction amino par des modes opératoires usuels,
puis, le cas échéant, on alkyle ou on dialkyle le groupe amino par voie de réduction avec un aldéhyde ou une cétone,
ou bien on l'alkyle ou on le dialkyle avec un halogénure,
puis on effectue le cas échant, en fonction des substituants indiqués ci-dessus, des dérivatisations par des procédés usuels tels que par exemple une alkylation ou une estérification.

5. Procédé de production de composés de formule générale (IIk) 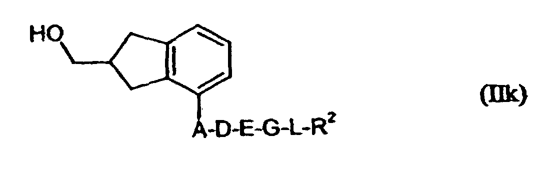 dans laquelle
A, D, E, G, L et R² ont la définition indiquée ci-dessus,
**caractérisé en ce qu'**on transforme des composés de formule générale (IIz) 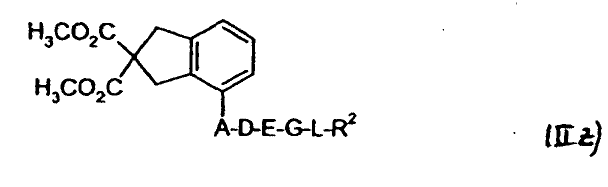 dans laquelle
A, D, E, G, L et R² ont la définition indiquée ci-dessus,
en utilisant HBr et l'acide acétique, en composés de formule générale (IIm) 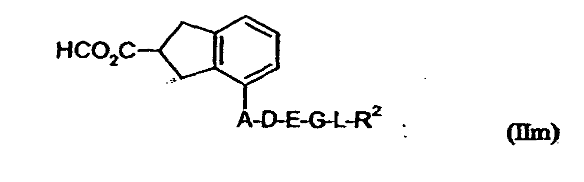 dans laquelle
A, D, E, G, L et R² ont la définition indiquée ci-dessus,
et on effectue dans une dernière étape une réduction avec BH₃ x S(CH₃)₂ dans le tétrahydrofuranne,
et dans le cas des énantiomères purs, on effectue une séparation par CLHP selon des modes opératoires classiques.

6. Préparations pharmaceutiques, qui contiennent comme constituant actif au moins un composé suivant l'une quelconque des revendications 1 à 3 en mélange avec au moins un support ou excipient non toxique acceptable du point de vue pharmaceutique.

7. Composés suivant l'une quelconque des revendications 1 à 3, destinés à être utilisés comme médicament dans le traitement d'êtres humains et d'animaux.

8. Utilisation des composés suivant l'une quelconque des revendications 1 à 3 pour la préparation d'un médicament destiné à la prévention et/ou au traitement de maladies neurodégénératives.

9. Utilisation des composés suivant l'une quelconque des revendications 1 à 3 pour la préparation d'un médicament destiné à la prévention et/ou au traitement d'ischémies cérébrales et de traumatismes crâne/cerveau.

10. Utilisation des composés suivant l'une quelconque des revendications 1 à 3 pour la préparation d'un médicament destiné au traitement d'états douloureux, d'émèse, de nausée, de glaucome, d'asthme, d'anorexie, de convulsions, de rhumatismes, de sédation et de troubles moteurs.

11. Utilisation des composés suivant l'une quelconque des revendications 1 à 3 pour la préparation d'un médicament destiné au traitement d'infections bactériennes ou virales, de maladies auto-immunes, de maladies d'origine inflammatoire ou auto-immunologiques des articulations de l'appareil osseux et musculaire, des organes internes et externes, du système nerveux central, des organes des sens et du système hématopoïétique chez l'homme et l'animal.

12. Utilisation des composés suivant l'une quelconque des revendications 1 à 3 pour la préparation d'un médicament destiné au traitement de la migraine et de la spasticité.
